(19) Europäisches Patentamt
European Patent Office
Office européen des brevets

(11) EP 0 824 521 B1

(12) EUROPÄISCHE PATENTSCHRIFT

(45) Veröffentlichungstag und Bekanntmachung des Hinweises auf die Patenterteilung:
**01.12.1999 Patentblatt 1999/48**

(21) Anmeldenummer: **96916034.0**

(22) Anmeldetag: **10.05.1996**

(51) Int Cl.6: **C07D 217/22**, C09K 19/34, C07D 401/04

(86) Internationale Anmeldenummer:
**PCT/EP96/01990**

(87) Internationale Veröffentlichungsnummer:
**WO 96/35673 (14.11.1996 Gazette 1996/50)**

(54) **1-FLUOR-6,7-DIHYDRO-5H-ISOCHINOLIN-8-ON-DERIVATE UND IHRE VERWENDUNG IN FLÜSSIGKRISTALLINEN MISCHUNGEN**

1-FLUOR-6,7-DIHYDRO-5H-ISOQUINOLINE-8-ONE-DERIVATIVES AND THE USE THEREOF IN LIQUID CRYSTALLINE MIXTURES

DERIVES DE 1-FLUORO-6,7-DIHYDRO-5H-ISOQUINOLEIN-8-ONE ET LEUR UTILISATION DANS DES MELANGES A CRISTAUX LIQUIDES

(84) Benannte Vertragsstaaten:
**DE FR GB**

(30) Priorität: **10.05.1995 DE 19517051**

(43) Veröffentlichungstag der Anmeldung:
**25.02.1998 Patentblatt 1998/09**

(73) Patentinhaber: **Aventis Research & Technologies GmbH & Co. KG**
**65926 Frankfurt am Main (DE)**

(72) Erfinder:
- **MANERO, Javier**
  **D-65931 Frankfurt am Main (DE)**
- **WINGEN, Rainer**
  **D-65795 Hattersheim (DE)**

(74) Vertreter: **Bardehle, Heinz, Dipl.-Ing. et al**
**Patent- und Rechtsanwälte**
**Bardehle . Pagenberg . Dost . Altenburg . Geissler . Isenbruck**
**Theodor-Heuss-Anlage 12**
**68165 Mannheim (DE)**

(56) Entgegenhaltungen:
**EP-A- 0 643 119** **WO-A-92/11241**

- **FERROELECTRICS (FEROA8,00150193);93; VOL.148 (1-4); PP.139-45, NIKKO KYODO CO., LTD.;PET. LAB.; TODA; 335; JAPAN (JP), XP000578754 YOKOYAMA A ET AL: "6-Alkyl-2-(4-alkyloxyphenyl)quinoline: a new smectic C base material***" in der Anmeldung erwähnt**

EP 0 824 521 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Neben nematischen und cholesterischen Flüssigkristallen werden in jüngerer Zeit auch optisch aktive, geneigt smektische (ferroelektrische) Flüssigkristalle in kommerziellen Displayvorrichtungen verwendet.

Clark und Lagerwall konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN ("twisted nematic")-Zellen um bis zu einem Faktor 1000 schnellere Schaltzeiten haben (siehe z.B. EP-A 0 032 362). Aufgrund dieser und anderer günstiger Eigenschaften, z.B. der bistabilen Schaltmöglichkeit und des nahezu blickwinkelunabhängigen Kontrasts, sind FLCs grundsätzlich für Anwendungsgebiete wie Computerdisplays gut geeignet.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle notig. Eine gute Orientierung in der $S_A$ und $S^*_C$-Phase läßt sich z.B. erreichen, wenn die Phasenfolge der Flüssigkristallmischung mit abnehmender Temperatur lautet:

$$\text{Isotrop} \rightarrow N^* \rightarrow S_A \rightarrow S^*_C$$

Vorraussetzung ist, daß der Pitch (Ganghöhe der Helix) in der N*-Phase sehr groß (größer 10 μm) oder, noch besser, völlig kompensiert ist (siehe z.B. T. Matsumoto et al., p. 468-470, Proc. of the 6th Int. Display Research Conf., Japan Display, Sept. 30 - Okto. 2, 1986, Tokyo, Japan; M. Murakami et al., ibid. S. 344 - S. 347). Dies erreicht man z. B., indem man zu der chiralen Flüssigkristallmischung, die in der N*-Phase z.B. eine linksdrehende Helix aufweist, einen oder mehrere optisch aktive Dotierstoffe, die eine rechtsdrehende Helix induzieren, in solchen Mengen hinzugibt, daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Voraussetzung, daß der Pitch in der smektischen C* Phase wesentlich größer ist als die Dicke des Anzeigeelementes (Mol. Cryst. Liq. Cryst. 94 (1983) 213-134 und 114 (1984) 151-187). Dies erreicht man, wie im Fall des cholesterischen Pitches, durch Verwendung von Dotierstoffen mit entgegengesetztem Drehsinn der Helix.

Die optische Schaltzeit $\tau$ [μs] ferroelektrischer Flüssigkristallsysteme, die möglichst kurz sein soll, hängt von der Rotationsviskosität des Systems $\gamma$ [mPas], der spontanen Polarisation $P_s$[nC/cm$^2$] und der elektrischen Feldstärke E[V/m] ab nach der Beziehung

$$\tau \sim \frac{\gamma}{P_s \bullet E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$, vorzugsweise $\approx 0{,}13$, und eine geringe positive oder vorzugsweise negative dielektrische Anisotropie $\Delta\varepsilon$ verlangt (siehe z.B. S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$- und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Ferroelektrische Flüssigkristallanzeigen lassen sich auch durch Nutzung des DHF (Distorted Helix Formation)-Effektes oder des PSFLCD-Effektes (Pitch Stabilized Ferroelectric Liquid Crystal Display, auch SBF = Short pitch Bistable Ferroelektric Effekt genannt) betreiben. Der DHF-Effekt wurde von B.I. Ostrovski in Advances in Liquid Crystal Research and Applications, Oxford/Budapest 1980, 469 ff. beschrieben, der PSFLCD-Effekt ist in DE-A 39 20 625 bzw. EP-A 0 405 346 beschrieben. Zur Nutzung dieser Effekte wird im Gegensatz zum SSFLCD-Effekt ein flüssigkristallines Material mit einem kurzen $S_C$-Pitch benötigt.

Chinolin- und Isochinolinderivate zur Verwendung in Flüssigkristallmischungen sind beispielsweise aus Ferroelectrics 148, 1993, 139-45 und der EP-A 0 643 119 bekannt.

Da die Entwicklung, insbesondere von ferroelektrischen Flüssigkristallmischungen, aber noch in keiner Weise als abgeschlossen betrachtet werden kann, sind die Hersteller von Displays an den unterschiedlichsten Komponenten für Mischungen interessiert. Dieses u.a. auch deshalb, weil erst das Zusammenwirken der flüssigkristallinen Mischungen mit den einzelnen Bauteilen der Anzeigevorrichtung bzw. der Zellen (z.B. der Orientierungsschicht) Rückschlüsse auf die Qualität auch der flüssigkristallinen Mischungen zuläßt.

Aufgabe der vorliegenden Erfindung war es daher, neue Verbindungen bereitzustellen, die in flüssigkristallinen Mischungen geeignet sind, das Eigenschaftsprofil dieser Mischungen zu verbessern.

Es wurde nun überraschend gefunden, daß 3,7-disubstituierte 1-Fluor-6,7-dihydro-5H-isochinolin-8-on-Derivate der Formel (I) in besonderer Weise zum Einsatz in Flüssigkristallmischungen geeignet sind.

Gegenstand der Erfindung sind daher Verbindungen der Formel (I),

$$R^1(-M^1)_a(-A^1-M^2)_b(-A^2-M^3)_c-B(-M^4-A^3)_d(-M^5-A^4)_e(-M^6)_f-R^2 \qquad (I)$$

in der die Symbole und Indizes folgende Bedeutungen haben:
die Gruppe B ist

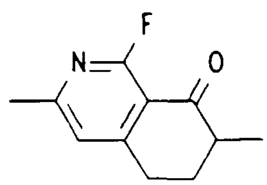

$R^1$, $R^2$ sind gleich oder verschieden Wasserstoff, -CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 20 C-Atomen (mit oder ohne asymmetrisches C-Atom), wobei auch eine oder mehrere $CH_2$-Gruppen durch -O-, -S-, -CO-O-, -O-CO-, -O-CO-O-, -CO-, -CS-, -CH =CH-, -C≡C-, Cyclopropan-1,2-diyl, $-Si(CH_3)_2-$, 1,4-Phenylen, trans-1,4-Cyclohexylen oder trans-1,3-Cyclopentylen ersetzt sein können, mit der Maßgabe, daß Sauerstoffatome und/oder Schwefelatome nicht unmittelbar miteinander gebunden sein dürfen, und/oder wobei ein oder mehrere H-Atome des Alkylrestes durch -F, -Cl, -Br, $-OR^3$, -SCN, -OCN oder $-N_3$ substituiert sein können, oder auch eine der nachfolgenden Gruppen (optisch aktiv oder racemisch):

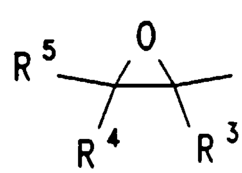
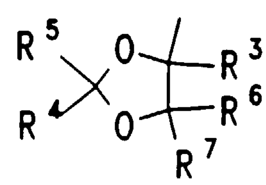
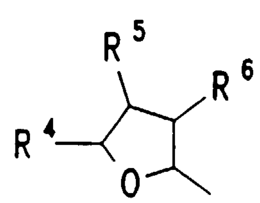

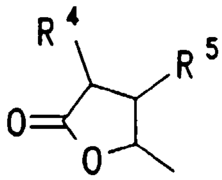
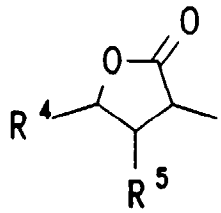
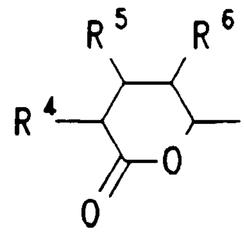

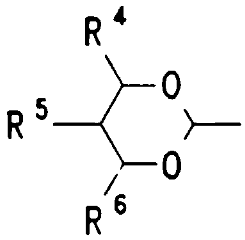
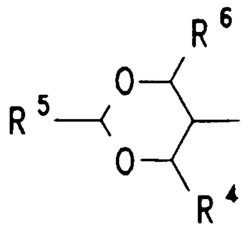
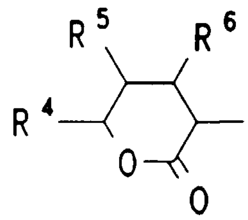

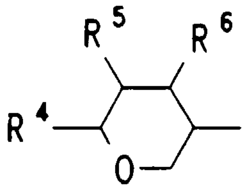
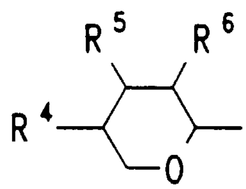
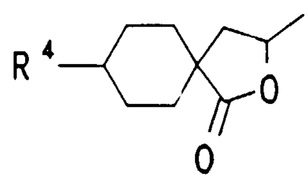

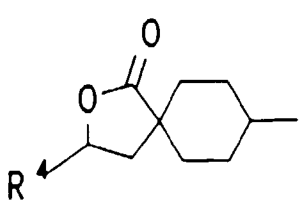
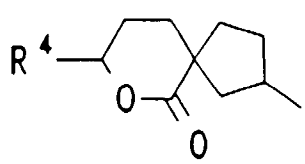
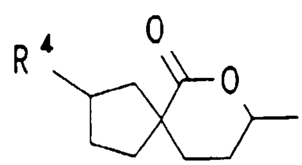

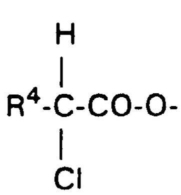
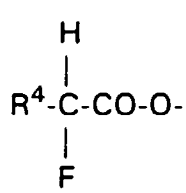
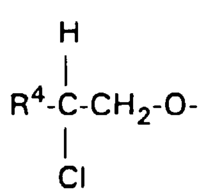
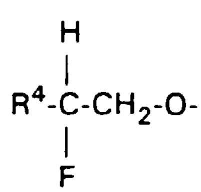

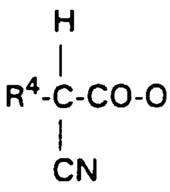 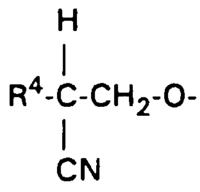 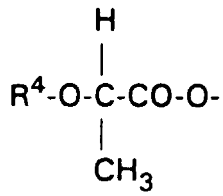 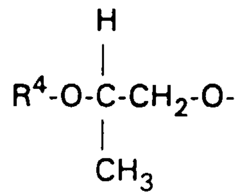

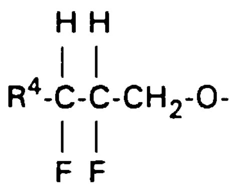 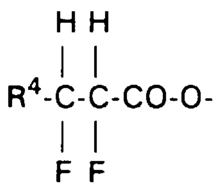

| | |
|---|---|
| $R^3$, $R^4$, $R^5$, $R^6$, $R^7$ | sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- und/oder -CH = CH- ersetzt sein können, mit der Maßgabe, daß Sauerstoffatome nicht unmittelbar miteinander gebunden sein dürfen, und/oder wobei ein oder mehrere H-Atome des Alkylrestes durch -F oder -Cl substituiert sein können; $R^4$ und $R^5$ können zusammen auch $-(CH_2)_4-$ oder $-(CH_2)_5-$ sein, wenn sie an ein Oxiran-, Dioxolan-, Tetrahydrofuran-, Tetrahydropyran-, Butyrolacton- oder Valerolacton-System gebunden sind; |
| $M^1$, $M^2$, $M^3$, $M^4$, $M^5$, $M^6$ | sind gleich oder verschieden -O-, -S-, -CO-, -CO-O-, -O-CO-, -O-CO-O-, -CO-S-, -S-CO-, -CS-O-, -O-CS-, -S-CS-S-, -O-CS-O, -S-CO-S-, -CS-, $-CH_2-O-$, $-O-CH_2-$, $-CH_2-S-$, $-S-CH_2-$, -CH=CH-, -C≡C-, $-CH_2-CH_2-CO-O-$, $-O-CO-CH_2-CH_2-$ oder eine Einfachbindung; |
| $A^1$, $A^2$, $A^3$, $A^4$ | sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, wobei ein oder zwei H-Atome durch CN und/oder $CH_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-2,5-diyl, wobei ein H-Atom durch F, Cl und/oder CN ersetzt sein kann, Thiophen-2,4-diyl, wobei ein H-Atom durch F, Cl und/oder CN ersetzt sein kann, Thiophen-2,5-diyl, wobei ein oder zwei H-Atome durch F, Cl und/oder CN ersetzt sein können, Piperazin-1,4-diyl, Piperazin-2,5-diyl, Naphthalin-2,6-diyl, wobei ein oder mehrere H-Atome durch F, Cl und/oder CN ersetzt sein können, Bicyclo[2.2.2]octan-1,4-diyl, wobei ein oder mehrere H-Atome durch F, Cl und/oder CN ersetzt sein können, 1,3-Dioxaborinan-2,5-diyl oder die Gruppe B; |
| a, b, c, d, e, f | sind null oder eins, mit der Maßgabe, daß die Summe aus b, c, d und e 0, 1 oder 2 sein muß. |

Die Verbindungen der Formel (I) sind in reinem Zustand farblos und bilden im allgemeinen flüssigkristalline Mesophasen in einem für die elektrooptische Verwendung günstig gelegenen Temperaturbereich. Chemisch, thermisch und gegen Licht sind sie stabil.

Besonders geeignet sind die Verbindungen der Formel (I), um schon in geringen Zumischmengen die dielektrische Anisotropie $\Delta\varepsilon$ flüssigkristalliner Mischungen in Richtung auf höhere negative Werte zu beeinflussen.

Bevorzugt sind solche Verbindungen der allgemeinen Formel (I), bei denen die Symbole und Indizes folgende Bedeutungen haben:

$R^1$, $R^2$ sind gleich oder verschieden Wasserstoff, -CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 18 C-Atomen (mit oder ohne asymmetrisches C-Atom), wobei auch eine oder mehrere $CH_2$-Gruppen durch -O-, -CO-, -CO-O-, -O-CO-, -O-CO-O-, -CH=CH-, -C≡C-, Cyclopropan-1,2-diyl, $-Si(CH_3)_2-$ oder trans-1,4-Cyclohexylen ersetzt sein können, mit der Maßgabe, daß Sauerstoffatome nicht unmittelbar miteinander gebunden sein dürfen, und/oder wobei ein oder mehrere H-Atome des Alkylrestes durch -F, -Cl, $-OR^3$, -OCN oder $-N_3$ substituiert sein können, oder eine der nachfolgenden Gruppen (optisch aktiv oder racemisch):

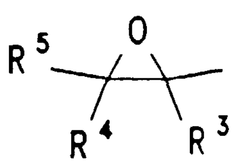
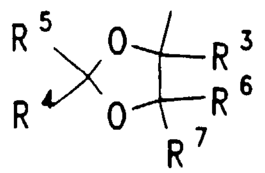
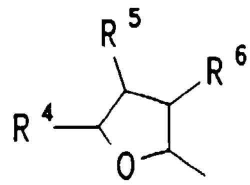

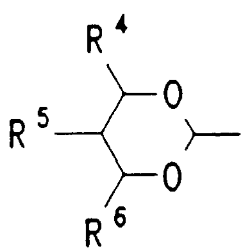
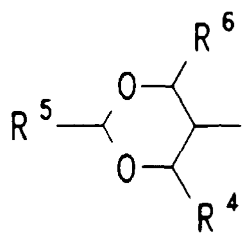
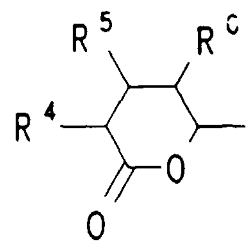

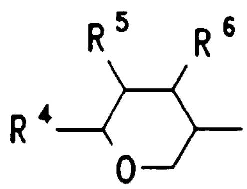
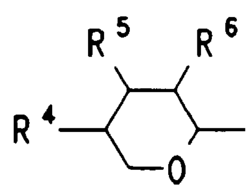
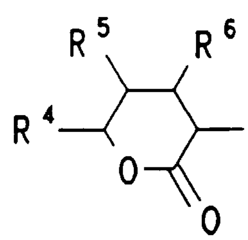

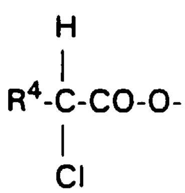
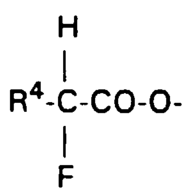
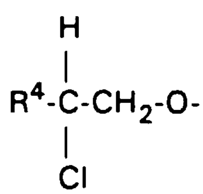
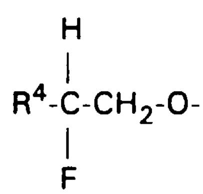

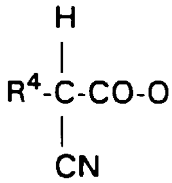 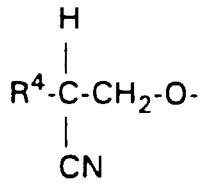 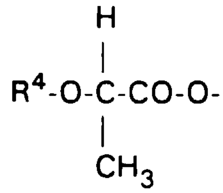 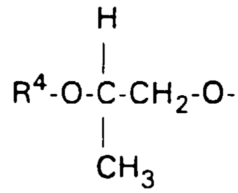

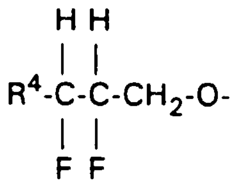 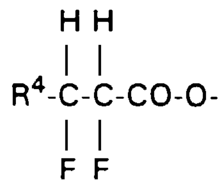

| | |
|---|---|
| $R^3$, $R^4$, $R^5$, $R^6$, $R^7$ | sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- und/oder -CH = CH-ersetzt sein können, mit der Maßgabe, daß Sauerstoffatome nicht unmittelbar miteinander gebunden sein dürfen, und/oder wobei ein oder mehrere H-Atome des Alkylrestes durch -F oder -Cl substituiert sein können; $R^4$ und $R^5$ können zusammen auch $-(CH_2)_4-$ oder $-(CH_2)_5-$ sein, wenn sie an ein Oxiran-, Dioxolan-, Tetrahydrofuran-, Tetrahydropyran- oder Valerolacton-System gebunden sind; |
| $M^1$, $M^2$, $M^3$, $M^4$, $M^5$, $M^6$ | sind gleich oder verschieden -O-, -CO-, -CO-O-, -O-CO-, -O-CO-O-, -O-CS-O-, $-CH_2-O-$, $-O-CH_2-$, -CH =CH-, -C≡C- oder eine Einfachbindung; |
| $A^1$, $A^2$, $A^3$, $A^4$ | sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, wobei ein oder zwei H-Atome durch CN und/oder $CH_3$ ersetzt sein können, (1,3,4)-Thiadiazol-2,5-diyl, 1,3-Dioxan-2,5-diyl, Thiophen-2,4-diyl, wobei ein H-Atom durch F, Cl und/oder CN ersetzt sein kann,Thiophen-2,5-diyl, wobei ein oder zwei H-Atome durch F, Cl und/oder CN ersetzt sein können, Naphthalin-2,6-diyl, wobei ein oder mehrere H-Atome durch F, Cl und/oder CN ersetzt sein können, oder die Gruppe B; |
| a, b, c, d, e, f | sind null oder eins, mit der Maßgabe, daß die Summe aus b, c, d und e 0, 1 oder 2 ist. |

Besonders bevorzugt sind solche Verbindungen der allgemeinen Formel (I), bei denen die Symbole und Indizes folgende Bedeutungen haben:

| | |
|---|---|
| $R^1$, $R^2$ | sind gleich oder verschieden Wasserstoff, -CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 16 C-Atomen (mit oder ohne asymmetrisches C-Atom), wobei auch eine, zwei oder drei $CH_2$-Gruppen durch -O-, -CO-, -O-CO-, -CO-O-, -CH=CH-, Cyclopropan-1,2-diyl, $-Si(CH_3)_2-$ oder trans-1,4-Cyclohexylen ersetzt sein können, mit der Maßgabe, daß Sauerstoffatome nicht unmittelbar miteinander gebunden sein dürfen, und/oder wobei ein oder mehrere H-Atome des Alkylrestes durch -F, -Cl oder $-OR^3$ substituiert sein können, oder auch eine der nachfolgenden Gruppen (optisch aktiv oder racemisch): |

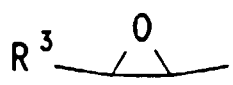
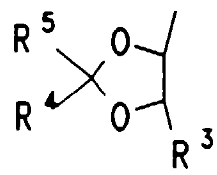
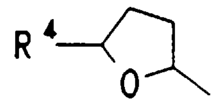

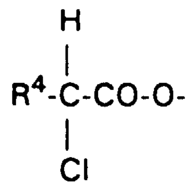
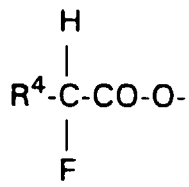
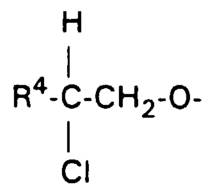
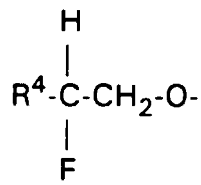

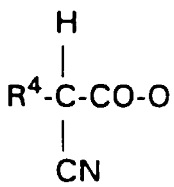
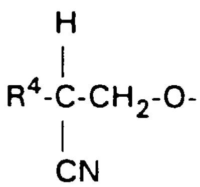
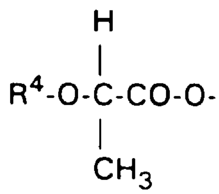
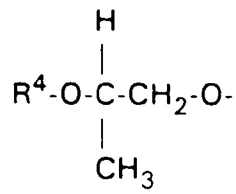

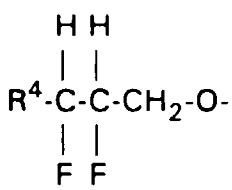
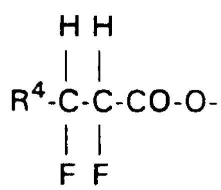

| | |
|---|---|
| $R^3$, $R^4$, $R^5$ | sind gleich oder verschieden Wasserstoff oder ein geradkettiger oder verzweigter Alkylrest mit 1-9 C-Atomen (mit oder ohne asymmetrisches C-Atom), wobei auch eine oder mehrere $CH_2$-Gruppen durch -O- und/oder -CH=CH- ersetzt sein können, mit der Maßgabe, daß Sauerstoffatome nicht unmittelbar miteinander gebunden sein dürfen, und/oder wobei ein oder mehrere H-Atome des Alkylrestes durch -F oder -Cl substituiert sein können; $R^4$ und $R^5$ können zusammen auch $-(CH_2)_4-$ oder $-(CH_2)_5-$ sein, wenn sie an ein Dioxolan-System gebunden sind; |
| $M^1$, $M^2$, $M^3$, $M^4$, $M^5$, $M^6$ | sind gleich oder verschieden -O-, -CO-, -CO-O-, -O-CO-, -O-CO-O-, $-CH_2-O-$, $-O-CH_2-$, -CH = CH- oder eine Einfachbindung; |
| $A^1$, $A^2$, $A^3$, $A^4$ | sind gleich oder verschieden 1,4-Phenylen, wobei ein, zwei oder drei H-Atome durch F, Cl und/oder CN ersetzt sein können, Pyridin-2,5-diyl, wobei ein oder zwei H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, wobei ein oder zwei H-Atome durch CN und/oder $CH_3$ ersetzt sein können, (1,3,4)-Thiadiazol-2,5-diyl oder Naphthalin-2,6-diyl, wobei ein oder zwei H-Atome durch F, Cl und/oder CN ersetzt sein können; |
| a, b, c, d, e, f | sind null oder eins, mit der Maßgabe, daß die Summe aus b, c, d und e 0, 1 oder 2 ist. |

Ganz besonders bevorzugt sind solche Verbindungen der allgemeinen Formel (I), bei denen die Symbole und Indizes folgende Bedeutungen haben:

$R^1$, $R^2$ sind gleich oder verschieden Wasserstoff oder ein geradkettiger oder verzweigter Alkylrest mit 1 bis 12 C-Atomen (mit oder ohne asymmetrisches C-Atom), wobei auch eine, zwei oder drei $CH_2$-Gruppen durch -O-, -O-CO-, -CO-O- oder trans-1,4-Cyclohexylen ersetzt sein können, mit der Maßgabe, daß Sauerstoffatome nicht unmittelbar miteinander gebunden sein dürfen, und/oder wobei ein oder mehrere H-Atome des Alkylrestes durch -F substituiert sein können, oder auch eine der nachfolgenden Gruppen (optisch aktiv oder racemisch):

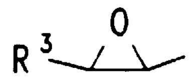

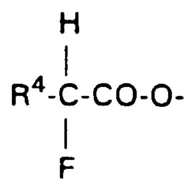

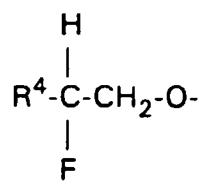

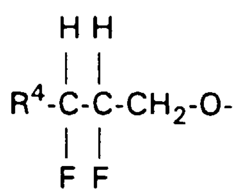

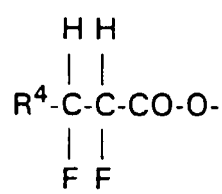

$R^3$, $R^4$ sind gleich oder verschieden Wasserstoff oder ein geradkettiger Alkylrest mit 1-9 C-Atomen, wobei auch eine oder mehrere $CH_2$-Gruppen durch -O- ersetzt sein können, mit der Maßgabe, daß Sauerstoffatome nicht unmittelbar miteinander gebunden sein dürfen;

$M^1$, $M^2$, $M^3$, $M^4$, $M^5$, $M^6$ sind gleich oder verschieden -O-, -CO-, -CO-O-, -O-CO-, -O-CO-O-, $-CH_2-O-$, $-O-CH_2-$, -CH=CH- oder eine Einfachbindung;

$A^1$, $A^2$, $A^3$, $A^4$ sind gleich oder verschieden 1,4-Phenylen, wobei ein, zwei oder drei H-Atome durch F ersetzt sein können, Pyridin-2,5-diyl, wobei ein oder zwei H-Atome durch F ersetzt sein können, Pyrimidin-2,5-diyl, trans-1,4-Cyclohexylen, wobei ein oder zwei H-Atome durch CN und/oder $CH_3$ ersetzt sein können oder Naphthalin-2,6-diyl, wobei ein oder zwei H-Atome durch F ersetzt sein können;

a, b, c, d, e, f sind null oder eins, mit der Maßgabe, daß die Summe aus b, c, d und e 0, 1 oder 2 ist.

Insbesondere bevorzugt sind Verbindungen der Formel (I), in denen die Gruppe $(-A^1-M^2)_b(-A^2-M^3)_c-B-(M^4-A^3)_d(-M^5-A^4)_e$ eine der folgenden Bedeutungen hat:

| | | |
|---|---|---|
| -Phe-Phe-(F)ICH | -Phe-Pym-(F)ICH | -Phe-Pyr-(F)ICH |
| -Phe-Diox-(F)ICH | -Phe-Naf-(F)ICH | $-Phe-F_2Phe-(F)ICH$ |
| -Phe-(F)Pyr-(F)ICH | -Phe-(F)Phe-(F)ICH | -Phe-TDZ-(F)ICH |

(fortgesetzt)

| | | |
|---|---|---|
| -Pym-Phe-(F)ICH | -Pym-Pym-(F)ICH | -Pym-Pyr-(F)ICH |
| -Pym-Diox-(F)ICH | -Pym-Naf-(F)ICH | -Pym-$F_2$Phe-(F)ICH |
| -Pym-(F)Pyr-(F)ICH | -Pym-(F)Phe-(F)ICH | -Pym-TDZ-(F)ICH |
| -Pyr-Phe-(F)ICH | -Pyr-Pym-(F)ICH | -Pyr-Pyr-(F)ICH |
| -Pyr-Diox-(F)ICH | -Pyr-Naf-(F)ICH | -Pyr-$F_2$Phe-(F)ICH |
| -Pyr-(F)Pyr-iF)ICH | -Pyr-(F)Phe-(F)ICH | -Pyr-TDZ-(F)ICH |
| -Diox-Phe-(F)ICH | -Diox-Pym-(F)ICH | -Diox-Pyr-(F)ICH |
| -Diox-Diox-(F)ICH | -Diox-Naf-(F)ICH | -Diox-$F_2$Phe-(F)ICH |
| -Diox-(F)Pyr-(F)ICH | -Diox-(F)Phe-(F)ICH | -Diox-TDZ-(F)ICH |
| -Naf-Phe-(F)ICH | -Naf-Pym-(F)ICH | -Naf-Pyr-(F)ICH |
| -Naf-Diox-(F)ICH | -Naf-Naf-(F)ICH | -Naf-$F_2$Phe-(F)ICH |
| -Naf-(F)Pyr-(F)ICH | -Naf-(F)Phe-(F)ICH | -Naf-TDZ-(F)ICH |
| -$F_2$Phe-Phe-(F)ICH | -$F_2$Phe-Pym-(F)ICH | -$F_2$Phe-Pyr-(F)ICH |
| -$F_2$Phe-Diox-(F)ICH | -$F_2$Phe-Naf-(F)ICH | -$F_2$Phe-$F_2$Phe-(F)ICH |
| -$F_2$Phe-(F)Pyr-(F)ICH | -$F_2$Phe-(F)Phe-(F)ICH | -$F_2$Phe-TDZ-(F)ICH |
| -(F)Pyr-Phe-(F)ICH | -(F)Pyr-Pym-(F)ICH | -(F)Pyr-Pyr-(F)ICH |
| -(F)Pyr-Diox-(F)ICH | -(F)Pyr-Naf-(F)ICH | -(F)Pyr-$F_2$Phe-(F)ICH |
| -(F)Pyr-(F)Pyr-(F)ICH | -(F)Pyr-(F)Phe-(F)ICH | -(F)Pyr-TDZ-(F)ICH |
| -(F)Phe-Phe-(F)ICH | -(F)Phe-Pym-(F)ICH | -(F)Phe-Pyr-(F)ICH |
| -(F)Phe-Diox-(F)ICH | -(F)Phe-Naf-(F)ICH | -(F)Phe-$F_2$Phe-(F)ICH |
| -(F)Phe-(F)Pyr-(F)ICH | -(F)Phe-(F)Phe-(F)ICH | -(F)Phe-TDZ-(F)ICH |
| -TDZ-Phe-(F)ICH | -TDZ-Pym-(F)ICH | -TDZ-Pyr-(F)ICH |
| -TDZ-Diox-(F)ICH | -TDZ-Naf-(F)ICH | -TDZ-$F_2$Phe-(F)ICH |
| -TDZ-(F)Pyr-(F)ICH | -TDZ-(F)Phe-(F)ICH | -TDZ-TDZ-(F)ICH |
| -Phe-(F)ICH | -Pym-(F)ICH | -Pyr-(F)ICH |
| -Diox-(F)ICH | -Naf-(F)ICH | -$F_2$Phe-(F)ICH |
| -(F)Pyr-(F)ICH | -(F)Phe-(F)ICH | -TDZ-(F)ICH |
| -(F)ICH-Phe-Phe | -(F)ICH-Pym-Phe | -(F)ICH-Pyr-Phe |
| -(F)ICH-Diox-Phe | -(F)ICH-Naf-Phe | -(F)ICH-$F_2$Phe-Phe |
| -(F)ICH-(F)Pyr-Phe | -(F)ICH-(F)Phe-Phe | -(F)ICH-TDZ-Phe |
| -(F)ICH-Phe-Pym | -(F)ICH-Pym-Pym | -(F)ICH-Pyr-Pym |
| -(F)ICH-Diox-Pym | -(F)ICH-Naf-Pym | -(F)ICH-$F_2$Phe-Pym |
| -(F)ICH-(F)Pyr-Pym | -(F)ICH-(F)Phe-Pym | -(F)ICH-TDZ-Pym |
| -(F)ICH-Phe-Pyr | -(F)ICH-Pym-Pyr | -(F)ICH-Pyr-Pyr |
| -(F)ICH-Diox-Pyr | -(F)ICH-Naf-Pyr | -(F)ICH-$F_2$Phe-Pyr |
| -(F)ICH-(F)Pyr-Pyr | -(F)ICH-(F)Phe-Pyr | -(F)ICH-TDZ-Pyr |
| -(F)ICH-Phe-Diox | -(F)ICH-Pym-Diox | -(F)ICH-Pyr-Diox |
| -(F)ICH-Diox-Diox | -(F)ICH-Naf-Diox | -(F)ICH-$F_2$Phe-Diox |
| -(F)ICH-(F)Pyr-Diox | -(F)ICH-(F)Phe-Diox | -(F)ICH-TDZ-Diox |
| -(F)ICH-Phe-Naf | -(F)ICH-Pym-Naf | -(F)ICH-Pyr-Naf |
| -(F)ICH-Diox-Naf | -(F)ICH-Naf-Naf | -(F)ICH-$F_2$Phe-Naf |
| -(F)ICH-(F)Pyr-Naf | -(F)ICH-(F)Phe-Naf | -(F)ICH-TDZ-Naf |
| -(F)ICH-Phe-$F_2$Phe | -(F)ICH-Pym-$F_2$Phe | -(F)ICH-Pyr-$F_2$Phe |
| -(F)ICH-Diox-$F_2$Phe | -(F)ICH-Naf-$F_2$Phe | -(F)ICH-$F_2$Phe-$F_2$Phe |
| -(F)ICH-(F)Pyr-$F_2$Phe | -(F)ICH-(F)Phe-$F_2$Phe | -(F)ICH-TDZ-$F_2$Phe |
| -(F)ICH-Phe-(F)Pyr | -(F)ICH-Pym-(F)Pyr | -(F)ICH-Pyr-(F)Pyr |
| -(F)ICH-Diox-(F)Pyr | -(F)ICH-Naf-(F)Pyr | -(F)ICH-$F_2$Phe-(F)Pyr |
| -(F)ICH-(F)Pyr-(F)Pyr | -(F)ICH-(F)Phe-(F)Pyr | -(F)ICH-TDZ-(F)Pyr |
| -(F)ICH-Phe-(F)Phe | -(F)ICH-Pym-(F)Phe | -(F)ICH-Pyr-(F)Phe |
| -(F)ICH-Diox-(F)Phe | -(F)ICH-Naf-(F)Phe | -(F)ICH-$F_2$Phe-(F)Phe |
| -(F)ICH-(F)Pyr-(F)Phe | -(F)ICH-(F)Phe-(F)Phe | -(F)ICH-TDZ-(F)Phe |

(fortgesetzt)

| | | |
|---|---|---|
| -(F)ICH-Phe-TDZ | -(F)ICH-Pym-TDZ | -(F)ICH-Pyr-TDZ |
| -(F)ICH-Diox-TDZ | -(F)ICH-Naf-TDZ | -(F)ICH-$F_2$Phe-TDZ |
| -(F)ICH-(F)Pyr-TDZ | -(F)ICH-(F)Phe-TDZ | -(F)ICH-TDZ-TDZ |
| -(F)ICH-Phe | -(F)ICH-Pym | -(F)ICH-Pyr |
| -(F)ICH-Diox | -(F)ICH-Naf | -(F)ICH-$F_2$Phe |
| -(F)ICH-(F)Pyr | -(F)ICH-(F)Phe | -(F)ICH-TDZ |
| -Phe-(F)ICH-Phe | -Pym-(F)ICH-Phe | -Pyr-(F)ICH-Phe |
| -Diox-(F)ICH-Phe | -Naf-(F)ICH-Phe | -$F_2$Phe-(F)ICH-Phe |
| -(F)Pyr-(F)ICH-Phe | -(F)Phe-(F)ICH-Phe | -TDZ-(F)ICH-Phe |
| -Phe-(F)ICH-Pym | -Pym-(F)ICH-Pym | -Pyr-(F)ICH-Pym |
| -Diox-(F)ICH-Pym | -Naf-(F)ICH-Pym | -$F_2$Phe-(F)ICH-Pym |
| -(F)Pyr-(F)ICH-Pym | -(F)Phe-(F)ICH-Pym | -TDZ-(F)ICH-Pym |
| -Phe-(F)ICH-Pyr | -Pym-(F)ICH-Pyr | -Pyr-(F)ICH-Pyr |
| -Diox-(F)ICH-Pyr | -Naf-(F)ICH-Pyr | -$F_2$Phe-(F)ICH-Pyr |
| -(F)Pyr-(F)ICH-Pyr | -(F)Phe-(F)ICH-Pyr | -TDZ-(F)ICH-Pyr |
| -Phe-(F)ICH-Diox | -Pym-(F)ICH-Diox | -Pyr-(F)ICH-Diox |
| -Diox-(F)ICH-Diox | -Naf-(F)ICH-Diox | -$F_2$Phe-(F)ICH-Diox |
| -(F)Pyr-(F)ICH-Diox | -(F)Phe-(F)ICH-Diox | -TDZ-(F)ICH-Diox |
| -Phe-(F)ICH-Naf | -Pym-(F)ICH-Naf | -Pyr-(F)ICH-Naf |
| -Diox-(F)ICH-Naf | -Naf-(F)ICH-Naf | -$F_2$Phe-(F)ICH-Naf |
| -(F)Pyr-(F)ICH-Naf | -(F)Phe-(F)ICH-Naf | -TDZ-(F)ICH-Naf |
| -Phe-(F)ICH-$F_2$Phe | -Pym-(F)ICH-$F_2$Phe | -Pyr-(F)ICH-$F_2$Phe |
| -Diox-(F)ICH-$F_2$Phe | -Naf-(F)ICH-$F_2$Phe | -$F_2$Phe-(F)ICH-$F_2$Phe |
| -(F)Pyr-(F)ICH-$F_2$Phe | -(F)Phe-(F)ICH-$F_2$Phe | -TDZ-(F)ICH-$F_2$Phe |
| -Phe-(F)ICH-(F)Pyr | -Pym-(F)ICH-(F)Pyr | -Pyr-(F)ICH-(F)Pyr |
| -Diox-(F)ICH-(F)Pyr | -Naf-(F)ICH-(F)Pyr | -$F_2$Phe-(F)ICH-(F)Pyr |
| -(F)Pyr-(F)ICH-(F)Pyr | -(F)Phe-(F)ICH-(F)Pyr | -TDZ-(F)ICH-(F)Pyr |
| -Phe-(F)ICH-(F)Phe | -Pym-(F)ICH-(F)Phe | -Pyr-(F)ICH-(F)Phe |
| -Diox-(F)ICH-(F)Phe | -Naf-(F)ICH-(F)Phe | -$F_2$Phe-(F)ICH-(F)Phe |
| -(F)Pyr-(F)ICH-(F)Phe | -(F)Phe-(F)ICH-(F)Phe | -TDZ-(F)ICH-(F)Phe |
| -Phe-(F)ICH-TDZ | -Pym-(F)ICH-TDZ | -Pyr-(F)ICH-TDZ |
| -Diox-(F)ICH-TDZ | -Naf-(F)ICH-TDZ | -$F_2$Phe-(F)ICH-TDZ |
| -(F)Pyr-(F)ICH-TDZ | -(F)Phe-(F)ICH-TDZ | -TDZ-(F)ICH-TDZ |

wobei die Abkürzungen

| | |
|---|---|
| (F)ICH | 1-Fluor-6,7-dihydro-5H-isochinolin-8-on-3,7-diyl, |
| Phe | 1,4-Phenylen, |
| Pyr | Pyridin-2,5-diyl, |
| Pym | Pyrimidin-2,5-diyl, |
| Diox | 1,3-Dioxan-2,5-diyl, |
| Naf | Naphthalin-2,6-diyl, |
| (F)Phe | Fluorbenzol-1,4-diyl, |
| $F_2$Phe | Difluorbenzol-1,4-diyl, |
| (F)Pyr | Fluorpyridin-2,5-diyl und |
| TDZ | (1,3,4)-Thiadiazol-2,5-diyl |

bedeuten und $M^1$, $M^6$, $R^1$, $R^2$ die in der Formel (I) angegebenen Bedeutungen haben.

Von diesen Gruppen sind bevorzugt:

-(F)ICH

-(F)ICH-Naf
-(F)ICH-Phe
-(F)ICH-Pym
-(F)ICH-Pyr
-Naf-(F)ICH
-Naf-(F)ICH-Naf
-Naf-(F)ICH-Phe
-Naf-(F)ICH-Pym
-NAF-(F)ICH-Pyr
-Phe-(F)ICH
-Phe-(F)ICH-Naf
-Phe-(F)ICH-Phe
-Phe-(F)ICH-Pym
-Phe-(F)ICH-Pyr
-Pym-(F)ICH
-Pym-(F)ICH-Naf
-Pym-(F)ICH-Phe
-Pym-(F)ICH-Pym
-Pym-(F)ICH-Pyr
-Pyr-(F)ICH
-Pyr-(F)ICH-Naf
-Pyr-(F)ICH-Phe
-Pyr-(F)ICH-Pym
-Pyr-(F)ICH-Pyr

und besonders bevorzugt:

-(F)ICH
-(F)ICH-Naf
-(F)ICH-Phe
-(F)ICH-Pym
-(F)ICH-Pyr
-Naf-(F)ICH
-Phe-(F)ICH
-Pym-(F)ICH
-Pyr-(F)ICH

Die Herstellung der erfindungsgemäßen Verbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.
Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.
Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.
Beispielhaft ist in Schema 1 ein Syntheseweg zu Verbindungen der Formel (I) angegeben, wobei auch andere Verfahren denkbar und möglich sind.

Schema 1:

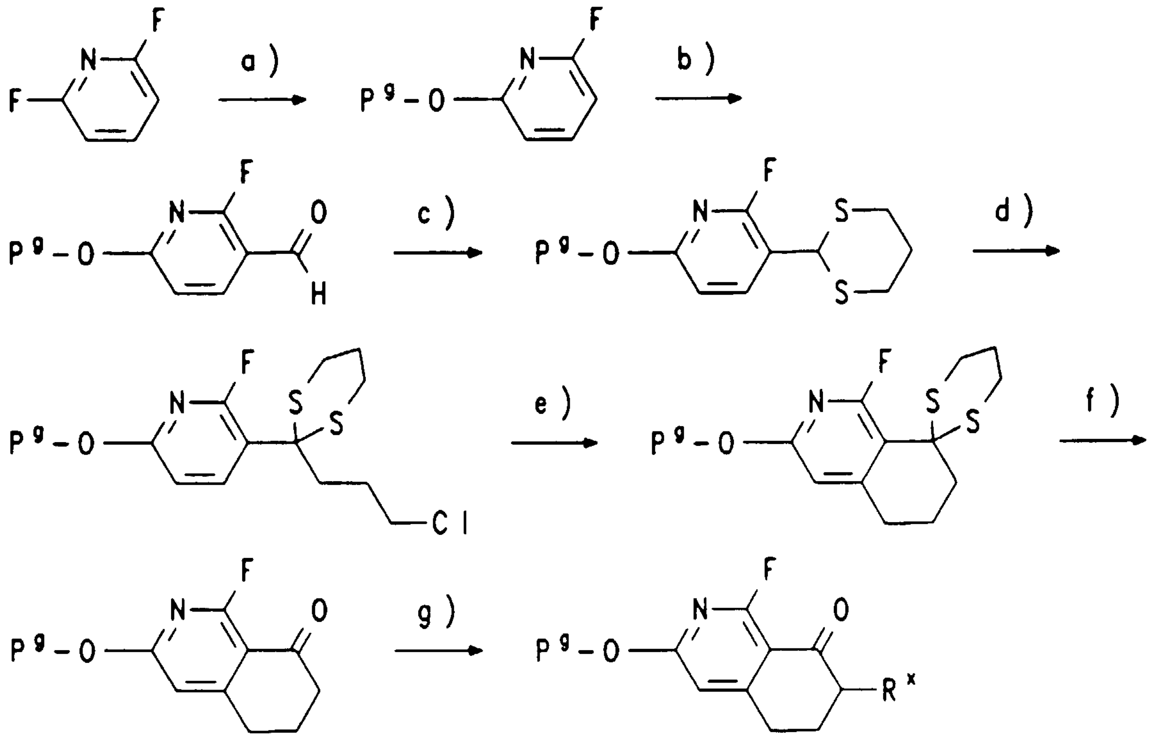


a) Base, $P^g$-OH;
b) 1. LDA (Lithiumdiisopropylamid); 2. DMF (N,N-Dimethylformamid); 3. $H^+$; analog J. Org. Chem. 51 (1986) 3762;
c) 1,3-Dimercaptopropan, $H^+$; analog Synthesis 1969, 17;
d) 1. BuLi; 2. 1-Chlor-3-iodopropan; analog J. Org. Chem. 33 (1068) 300;
e) z.B. $AlCl_3$;
f) z.B. $HgCl_2$/MeOH; analog Chem. Reviews 49 (1950) 67;
g) 1. Base; 2. $R^x$-X; analog Rec. Chem. Prog. 28 (1968) 99.

Die Gruppe $P^g$ ist die Gruppierung $R^1(-M^1)_a(-A^1-M^2)_b(-A^2-M^3)_c$ oder eine geeignete, gegebenenfalls geschützte Vorstufe hiervon, die in späteren Schritten nach an sich bekannten, dem Fachmann geläufigen Methoden in diese Gruppierung überführt werden kann.

Beispielsweise kann $P^g$ ein Perfluoralkylsulfonat sein, wobei dann durch Kupplung mit beispielsweise einer entsprechenden Boronsäure die Gruppierung $R^1(-M^1)_a(-A^1-M^2)_b(-A^2-M^3)_c$ eingeführt wird.

$R^x$ ist die Gruppierung $(-M^4-A^3)_d(-M^5-A^4)_e(-M^6)_f-R^2$ oder eine geeignete, gegebenenfalls geschützte Vorstufe hiervon, die in späteren Schritten nach an sich bekannten, dem Fachmann geläufigen Methoden in diese Gruppierung überführt werden kann.

Die Synthese des Restes $R^1(-M^1)_a(-A^1-M^2)_b(-A^2-M^3)_c$ bzw. $(-M^4-A^3)_d(-M^5-A^4)_e(-M^6)_f-R^2$ erfolgt nach an sich bekannten, dem Fachmann geläufigen Methoden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Beispielsweise sei verwiesen auf DE-A 23 44 732, 24 50 088, 24 29 093, 25 02 94, 26 36 684, 27 01 591 und 27 52 975 für Verbindungen mit 1,4-Cyclohexylen und 1,4-Phenylen-Gruppen; DE-A 26 41 724 für Verbindungen mit Pyrimidin-2,5-diyl-Gruppen; DE-A 40 26 223 und EP-A 03 91 203 für Verbindungen mit Pyridin-2,5-diyl-Gruppen; DE-A 32 31 462 für Verbindungen mit Pyridazin-3,6-diyl-Gruppen; EP-A 309 514 für Verbindungen mit (1,3,4)-Thiadiazol-2-5-diyl-Gruppen; WO-A 92/16500 für Naphthalin-2,6-diyl-Gruppen; DE-A 37 10 890 für Bicyclo[2.2.2.]octan-1,4-diyl-Gruppen; K. Seto et al, Journal of the Chemical Society, Chemical Communications 1988, 56 für Dioxoborinan-2,5-diyl-Gruppen.

Die Herstellung disubstituierter Pyridine, disubstituierter Pyrazine, disubstituierter Pyrimidine und disubstituierter Pyridazine findet sich beispielsweise auch in den entsprechenden Bänden der Serie "The Chemistry of Heterocyclic Compounds" von A. Weissberger und E.C. Taylor (Herausgeber).

Dioxanderivate werden zweckmäßig durch Reaktion eines entsprechenden Aldehyds (oder eines seiner reaktionsfähigen Derivate) mit einem entsprechenden 1,3-Diol (oder einem seiner reaktionsfähigen Derivate) hergestellt, vorzugsweise in Gegenwart eines inerten Lösungsmittels, wie Benzol oder Toluol, und/oder eines Katalysators, z.B. einer starken Säure, wie Schwefelsäure, Benzol- oder p-Toluolsulfonsäure, bei Temperaturen zwischen etwa 20°C und etwa 150°C, vorzugsweise zwischen 80°C und 120°C. Als reaktionsfähige Derivate der Ausgangsstoffe eignen sich in erster Linie Acetale.

Die genannten Aldehyde und 1,3-Diole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester erhältlich.

Verbindungen, worin ein aromatischer Ring durch mindestens ein F-Atom substituiert ist, können auch aus den entsprechenden Diazoniumsalzen durch Austausch der Diazoniumgruppe gegen ein Fluoratom, z.B. nach den Methoden von Balz und Schiemann, erhalten werden.

Was die Verknüpfung der Ringsysteme miteinander angeht, sei beispielsweise verwiesen auf:
N. Miyaura, T. Yanagai und A. Suzuki in Synthetic Communications 11 (1981), 513-519; DE-C 39 30 663; M.J. Sharp, W. Cheng, V. Snieckus in Tetrahedron Letters 28 (1987) 5093; G.W. Gray in J. Chem. Soc. Perkin Trans II 1989, 2041 und Mol. Cryst. Liq. Cryst. 172 (1989) 165, 204 (1991) 43 und 91; EP-A 0 449 015; WO-A 89/12039; WO-A 89/03821; EP-A 0 354 434 für die direkte Verknüpfung von Aromaten und Heteroaromaten; DE-A 32 01 721 für Verbindungen mit $-CH_2CH_2-$Brückengliedern und Koji Seto et al. in Liquid Crystals 8 (1990) 861-870 für Verbindungen mit $-C{\equiv}C-$Brückengliedern.

Ester der Formel (I) können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt und können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls, wie Natrium oder Kalium, in Betracht.

Die Veresterung wird vorteilhaft in Gegenwart eines inerten Lösungsmittels durchgeführt. Gut geeignet sind insbesondere Ether, wie Diethylether, Di-n-butylether, THF (Tetrahydrofuran), Dioxan oder Anisol, Ketone, wie Aceton, Butanon oder Cyclohexanon, Amide, wie DMF oder Phosphorsäurehexamethyltriamid, Kohlenwasserstoffe, wie Benzol, Toluol oder Xylol, Halogenkohlenwasserstoffe, wie Tetrachlorkohlenstoff, Dichlormethan oder Tetrachlorethylen und Sulfoxide, wie Dimethylsulfoxid oder Sulfolan.

Ether der Formel (I) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, Alkylsulfonat oder Dialkylsulfat umgesetzt werden, zweckmäßig in einem inerten Lösungsmittel, wie Aceton, 1,2-Dimethoxyethan, DMF oder Dimethylsulfoxid, oder auch mit einem Überschuß an wäßriger oder wäßrig-alkoholischer NaOH oder KOH bei Temperaturen zwischen etwa 20° und 100°C.

Was die Synthese spezieller Reste $R^1$ angeht, sei zusätzlich beispielsweise verwiesen auf EP-A 0 355 008 für Verbindungen mit siliziumhaltigen Seitenketten und EP-A 0 292 954 und EP-A 0 398 155 für Verbindungen mit Cyclopropylgruppen in der Seitenkette.

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

In diesem Zusammenhang besitzen die Verbindungen der Formel (I) einen breiten Anwendungsbereich. In Abhängigkeit von der Auswahl der Substituenten können sie als Basismaterialien dienen, aus denen flüssigkristalline Phasen zum überwiegenden Teil zusammengesetzt sind; es können aber auch Verbindungen der Formel (I) flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Gegenstand der Erfindung ist auch die Verwendung von Verbindungen der Formel (I) in Flüssigkristallmischungen, vorzugsweise ferroelektrischen und nematischen, insbesondere ferroelektrischen.

Weiterhin Gegenstand der Erfindung sind Flüssigkristallmischungen, vorzugsweise ferroelektrische und nematische, insbesondere ferroelektrische, enthaltend eine oder mehrere Verbindungen der Formel (I).
Die erfindungsgemäßen Flüssigkristallmischungen enthalten im allgemeinen 2 bis 35, vorzugsweise 2 bis 25, besonders bevorzugt 2 bis 20 Komponenten.
Sie enthalten im allgemeinen 0,01 bis 80 Gew.-%, vorzugsweise 0,1 bis 60 Gew.-%, besonders bevorzugt 0,1 bis 30 Gew.-%, an einer oder mehreren, vorzugsweise 1 bis 10, besonders bevorzugt 1 bis 5, ganz besonders bevorzugt 1 bis 3, der erfindungsgemäßen Verbindungen der Formel (I).
Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. Dazu gehören z. B.:

- Derivate des Phenylpyrimidins, wie beispielsweise in WO 86/06401, US-A 4 874 542 beschrieben,
- metasubstituierte Sechsringaromaten, wie beispielsweise in EP-A 0 578 054 beschrieben,
- Siliziumverbindungen, wie beispielsweise in EP-A 0 355 008 beschrieben,
- mesogene Verbindungen mit nur einer Seitenkette, wie beispielsweise in EP-A 0 541 081 beschrieben,
- Hydrochinonderivate, wie beispielsweise in EP-A 0 603 786 beschrieben,
- Pyridylpyrimidine, wie beispielsweise in WO 92/12974 beschrieben,
- Phenylbenzoate, wie beispielsweise bei P. Keller, Ferroelectrics 58 (1984), 3 und J. W. Goodby et al., Liquid Crystals and Ordered Fluids, Bd. 4, New York 1984 beschrieben und
- Thiadiazole, wie beispielsweise in EP-A 0 309 514 beschrieben.

Als chirale, nicht racemische Dotierstoffe kommen beispielsweise in Frage:

- optisch aktive Phenylbenzoate, wie beispielsweise bei P. Keller, Ferroelectrics 58 (1984), 3 und J. W. Goodby et al., Liquid Crystals and Ordered Fluids, Bd. 4, New York 1984 beschrieben,
- optisch aktive Oxiranether, wie beispielsweise in EP-A 0 263 437 und WO-A 93/13093 beschrieben,
- optisch aktive Oxiranester, wie beispielsweise in EP-A 0 292 954 beschrieben,
- optisch aktive Dioxolanether, wie beispielsweise in EP-A 0 351 746 beschrieben,
- optisch aktive Dioxolanester, wie beispielsweise in EP-A 0 361 272 beschrieben,
- optisch aktive Tetrahydrofuran-2-carbonsäureester, wie beispielsweise in EP-A 0 355 561 beschrieben, und
- optisch aktive 2-Fluoralkylether, wie beispielsweise in EP-A 0 237 007 und US-A 5,051,506 beschrieben.

Die Mischungen wiederum können Anwendung finden in elektrooptischen oder vollständig optischen Elementen, z. B. Anzeigeelementen, Schaltelementen, Lichtmodulatoren, Elementen zur Bildbearbeitung und/oder Signalverarbeitung oder allgemein im Bereich der nichtlinearen Optik.
Flüssigkristalline Mischungen, die Verbindungen der allgemeinen Formel (I) enthalten, sind besonders für die Verwendung in elektrooptischen Schalt- und Anzeigevorrichtungen (Displays) geeignet. Diese Displays sind üblicherweise so aufgebaut, daß eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z.B. aus Glas) sind. Darüberhinaus enthalten sie Abstandshalter, Kleberahmen, Polarisatoren sowie für Farbdisplays dünne Farbfilterschichten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z.B. E. Kaneko, "Liquid Crystal TV Displays: Principles and Applications of Liquid Crystal Displays", KTK Scientific Publishers 1987).
Ferner sind die Mischungen für Feldbehandlung, d. h. zum Betrieb in der Quasi-Bookshelf-Geometrie (QBG) (siehe z. B. H. Rieger et al., SID 91 Digest (Anaheim) 1991, 396) geeignet.
Ebenso sind die erfindungsgemäßen Mischungen geeignet für die Verwendung in ferroelektrischen Flüssigkristallanzeigen, die auf Nutzung des DHF-Effekts oder des PSFLCD-Effekts (Pitch Stabilized Ferroelectric Liquid Crystal Display, auch SBF = Short Pitch Bistable Ferroelectric Effect genannt) beruhen.
Daneben können die Verbindungen der Formel (I) auch als Komponenten von antiferroelektrischen Flüssigkristallmischungen Verwendung finden.
Auf die in dieser Anmeldung zitierten Literaturstellen wird ausdrücklich Bezug genommen; sie sind durch Zitat Bestandteil der Beschreibung.
Die Erfindung wird durch die Beispiele näher erläutert, ohne sie darauf beschränken zu wollen.

Beispiel 1:

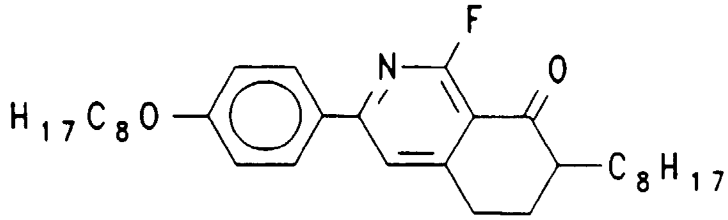

1-Fluor-3-[4-(octyloxy)-phenyl]-7-octyl-6,7-dihydro-5H-isochinolin-8-on

10 mmol 1-Fluor-3-(4-hydroxy-phenyl)-7-octyl-6,7-dihydro-5H-isochinolin-8-on werden in 50 ml DMF gelöst und mit 11 mmol Natriumhydrid versetzt. Nach 30 Minuten Rühren tropft man 11 mmol 1-Octylbromid zu, rührt noch 140 Minuten bei 60°C und gießt in Wasser. Das Gemisch wird mit Dichlormethan extrahiert, die vereinigten organischen Phasen werden getrocknet, das Solvens im Vakuum entfernt und der Rückstand an Kieselgel chromatographiert. Man erhält 8 mmol 1-Fluoro-3-[4-(octyloxy)-phenyl]-7-octyl-6,7-dihydro-5H-isochinolin-8-on.

Analog Beispiel 1 lassen sich 1-Fluor-3-(4-hydroxy-phenyl)-7-alkyl-6,7-dihydro-5H-isochinolin-8-one mit weiteren Alkylhalogeniden, Sulfonsäure-2-fluoralkylestern, Sulfonsäure-2,3-difluoralkylestern, Sulfonsäure-3-alkyl-oxiran-2-methylestern, (ω-Brom-alkyl)-cyclopropanen oder 1 -Brom-dimethylsilanylalkanen umsetzen.

Beispiel 2:

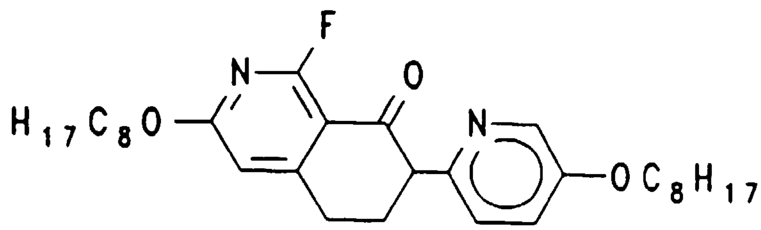

7-[5-(Octyloxy)-pyridin-2-yl]-1-fluor-3-octyloxy-6,7-dihydro-5H-isochinolin-8-on

10 mmol 1-Fluor-7-(5-hydroxy-pyridin-2-yl)-3-octyloxy-6,7-dihydro-5H-isochinolin-8-on werden in 50 ml DMF gelöst und mit 11 mmol Natriumhydrid versetzt. Nach 30 Minuten rühren tropft man 11 mmol 1-Octylbromid zu, rührt noch 140 Minuten bei 60°C und gießt in Wasser. Das Gemisch wird mit Dichlormethan extrahiert, die vereinigten organischen Phasen werden getrocknet, das Solvens im Vakuum entfernt und der Rückstand an Kieselgel chromatographiert. Man erhält 7,55 mmol 7-[5-(Octyloxy)-pyridin-2-yl]-1-fluor-3-octyloxy-6,7-dihydro-5H-isochinolin-8-on.

Analog Beispiel 2 lassen sich 1-Fluor-7-(5-hydroxy-pyridin-2-yl)-3-alkyloxy-6,7-dihydro-5H-isochinolin-8-one mit Sulfonsäure-2-fluoralkylestern, Sulfonsäure-2,3-difluoralkylestern, Sulfonsäure-3-alkyl-oxiran-2-methylestern, (ω-Brom-alkyl)-cyclopropanen oder 1-Brom-dimethylsilanylalkanen umsetzen.

Beispiel 3:

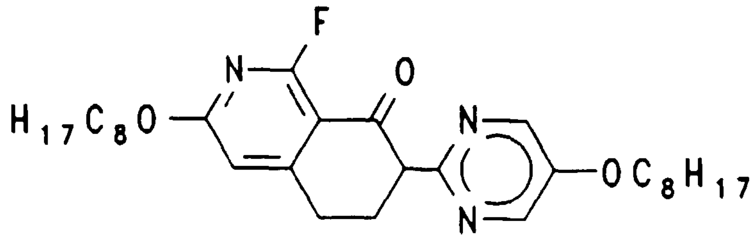

7-[5-(Octyloxy)-pyrimidin-2-yl]-1-fluor-3-octyloxy-6,7-dihydro-5H-isochinolin-8-on

10 mmol 1-Fluor-7-(5-hydroxy-pyrimidin-2-yl)-3-octyloxy-6,7-dihydro-5H-isochinolin-8-on werden in 50 ml DMF gelöst und mit 11 mmol Natriumhydrid versetzt. Nach 30 Minuten Rühren tropft man 11 mmol 1-Octylbromid zu, rührt noch 140 Minuten bei 60°C und gießt in Wasser. Das Gemisch wird mit Dichlormethan extrahiert, die vereinigten organischen Phasen werden getrocknet, das Solvens im Vakuum entfernt und der Rückstand an Kieselgel chromatographiert. Man erhält 9 mmol 7-[5-(Octyloxy)-pyrimidin-2-yl]-1-fluor-3-octyloxy-6,7-dihydro-5H-isochinolin-8-on.

Analog Beispiel 3 lassen sich 1-Fluor-7-(5-hydroxy-pyrimidin-2-yl)-3-alkyloxy-6,7-dihydro-5H-isochinolin-8-one mit Sulfonsäure-2-fluoralkylestern, Sulfonsäure-2,3-difluoralkylestern, Sulfonsäure-3-alkyl-oxiran-2-methylestern, (ω-Bromalkyl)-cyclopropanen oder 1-Brom-dimethylsilanylalkanen umsetzen.

## Patentansprüche

**1.** 1-Fluor-6,7-dihydro-5H-isochinolin-8-on Derivat der Formel (I),

$$R^1(-M^1)_a(-A^1-M^2)_b(-A^2-M^3)_c-B(-M^4-A^3)_d(-M^5-A^4)_e(-M^6)_f-R^2 \qquad (I)$$

in der die Symbole und Indizes folgende Bedeutungen haben:
die Gruppe B ist

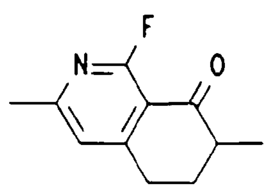

$R^1$, $R^2$ sind gleich oder verschieden Wasserstoff, -CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 20 C-Atomen (mit oder ohne asymmetrisches C-Atom), wobei auch eine oder mehrere $CH_2$-Gruppen durch -O-, -S-, -CO-O-, -O-CO-, -O-CO-O-, -CO-, -CS-, -CH=CH-, -C≡C-, Cyclopropan-1,2-diyl, $-Si(CH_3)_2-$, 1,4-Phenylen, trans-1,4-Cyclohexylen oder trans-1,3-Cyclopentylen ersetzt sein können, mit der Maßgabe, daß Sauerstoffatome und/oder Schwefelatome nicht unmittelbar miteinander gebunden sein dürfen, und/oder wobei ein oder mehrere H-Atome des Alkylrestes durch -F, -Cl, -Br, $-OR^3$, -SCN, -OCN oder $-N_3$ substituiert sein können, oder auch eine der nachfolgenden Gruppen (optisch aktiv oder racemisch):

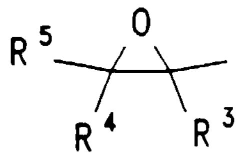
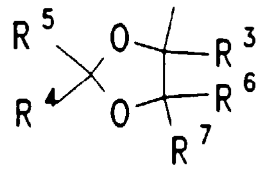
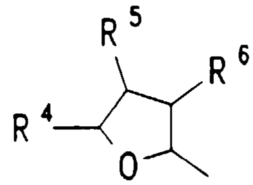

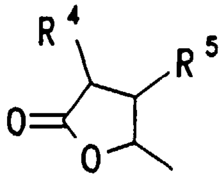
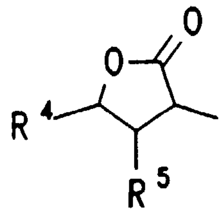
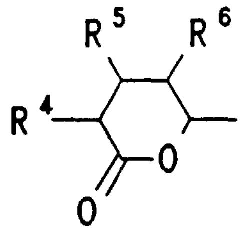

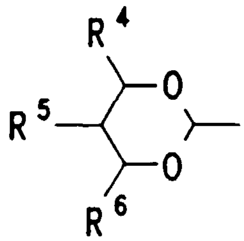
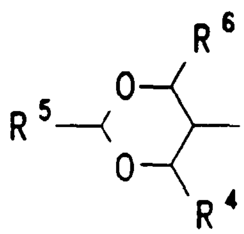
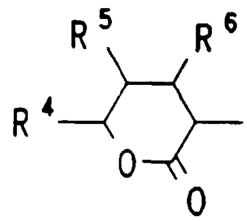

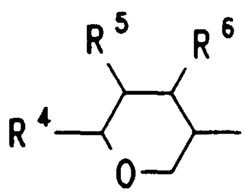
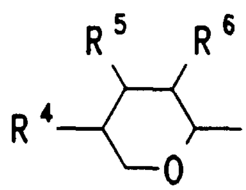
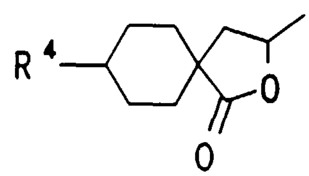

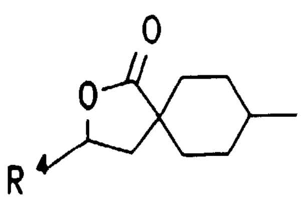
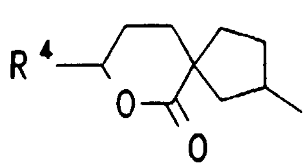
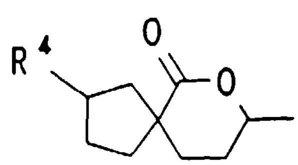

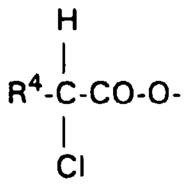
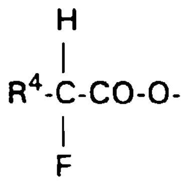
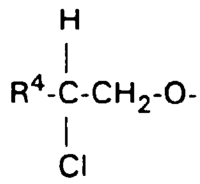
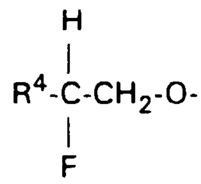

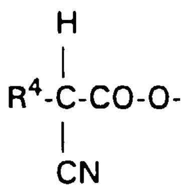
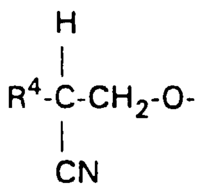
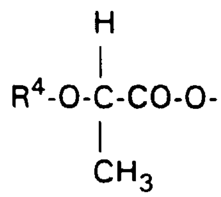
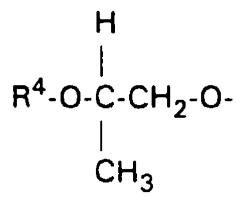

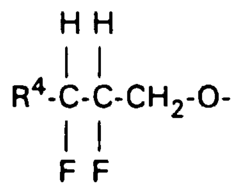
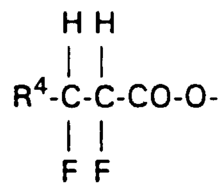

| | |
|---|---|
| $R^3$, $R^4$, $R^5$, $R^6$, $R^7$ | sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- und/oder -CH=CH- ersetzt sein können, mit der Maßgabe, daß Sauerstoffatome nicht unmittelbar miteinander gebunden sein dürfen, und/oder wobei ein oder mehrere H-Atome des Alkylrestes durch -F oder -Cl substituiert sein können; $R^4$ und $R^5$ können zusammen auch $-(CH_2)_4-$ oder $-(CH_2)_5-$ sein, wenn sie an ein Oxiran-, Dioxolan-, Tetrahydrofuran-, Tetrahydropyran-, Butyrolacton- oder Valerolacton-System gebunden sind; |
| $M^1$, $M^2$, $M^3$, $M^4$, $M^5$, $M^6$ | sind gleich oder verschieden -O-, -S-, -CO-, -CO-O-, -O-CO-, -O-CO-O-, -CO-S-, -S-CO-, -CS-O-, -O-CS-, -S-CS-S-, -O-CS-O, -S-CO-S-, -CS-, $-CH_2-O-$, $-O-CH_2-$, $-CH_2-S-$, $-S-CH_2-$, -CH=CH-, -C≡C-, $-CH_2-CH_2-CO-O-$, $-O-CO-CH_2-CH_2-$ oder eine Einfachbindung; |
| $A^1$, $A^2$, $A^3$, $A^4$ | sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, wobei ein oder zwei H-Atome durch CN und/oder $CH_3$ ersetzt sein können, (1,3,4)-Thiadiazol-2,5-diyl, 1,3-Dioxan-2,5-diyl, 1,3-Dithian-2,5-diyl, 1,3-Thiazol-2,4-diyl, wobei ein H-Atom durch F, Cl und/oder CN ersetzt sein kann, 1,3-Thiazol-2,5-diyl, wobei ein H-Atom durch F, Cl und/oder CN ersetzt sein kann, Thiophen-2,4-diyl, wobei ein H-Atom durch F, Cl und/oder CN ersetzt sein kann, Thiophen-2,5-diyl, wobei ein oder zwei H-Atome durch F, Cl und/oder CN ersetzt sein können, Piperazin-1,4-diyl, Piperazin-2,5-diyl, Naphthalin-2,6-diyl, wobei ein oder mehrere H-Atome durch F, Cl und/oder CN ersetzt sein können, Bicyclo[2.2.2]octan-1,4-diyl, wobei ein oder mehrere H-Atome durch F, Cl und/oder CN ersetzt sein können, 1,3-Dioxaborinan-2,5-diyl oder die Gruppe B; |

a, b, c, d, e, f — sind null oder eins, mit der Maßgabe, daß die Summe aus b, c, d und e 0, 1 oder 2 sein muß;

**2.** 1-Fluor-6,7-dihydro-5H-isochinolin-8-on-Derivate nach Anspruch 1, dadurch gekennzeichnet, daß die Symbole und Indizes in der Formel (I) folgende Bedeutungen haben:

$R^1$, $R^2$ — sind gleich oder verschieden Wasserstoff, -CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 18 C-Atomen (mit oder ohne asymmetrisches C-Atom), wobei auch eine oder mehrere $CH_2$-Gruppen durch -O-, -CO-, -CO-O-, -O-CO-, -O-CO-O-, -CH =CH-, -C≡C-, Cyclopropan-1,2-diyl, $-Si(CH_3)_2-$ oder trans-1,4-Cyclohexylen ersetzt sein können, mit der Maßgabe, daß Sauerstoffatome nicht unmittelbar miteinander gebunden sein dürfen, und/oder wobei ein oder mehrere H-Atome des Alkylrestes durch -F, -Cl, $-OR^3$, -OCN oder $-N_3$ substituiert sein können, oder eine der nachfolgenden Gruppen (optisch aktiv oder racemisch):

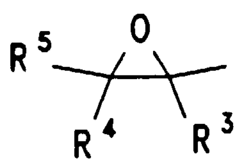
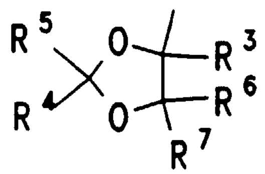
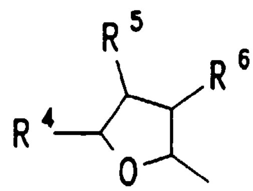

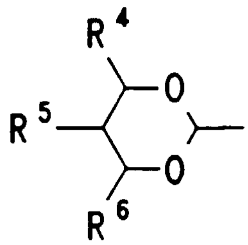
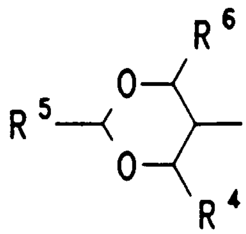
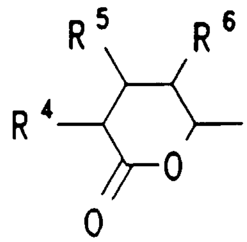

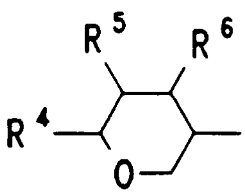
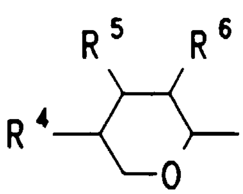
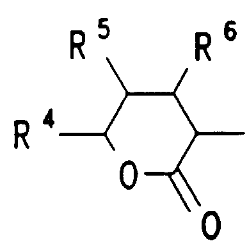

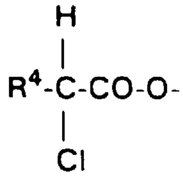
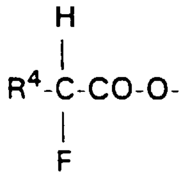
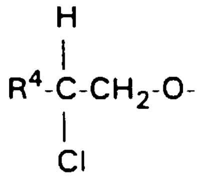
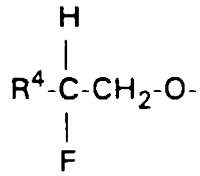

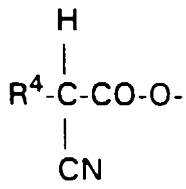 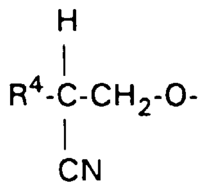 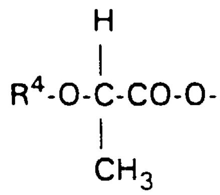 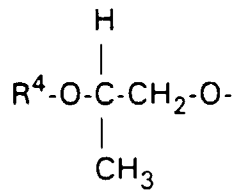

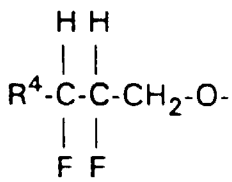 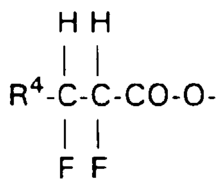

| | |
|---|---|
| $R^3$, $R^4$, $R^5$, $R^6$, $R^7$ | sind gleich oder verschieden Wasserstoff oder ein geradkettiger oder verzweigter Alkylrest mit 1-16 C-Atomen (mit oder ohne asymmetrisches C-Atom), wobei auch eine oder mehrere $CH_2$-Gruppen durch -O- und/oder -CH = CH- ersetzt sein können, mit der Maßgabe, daß Sauerstoffatome nicht unmittelbar miteinander gebunden sein dürfen, und/oder wobei ein oder mehrere H-Atome des Alkylrestes durch -F oder -Cl substituiert sein können; $R^4$ und $R^5$ können zusammen auch $-(CH_2)_4-$ oder $-(CH_2)_5-$ sein, wenn sie an ein Oxiran-, Dioxolan-, Tetrahydrofuran-, Tetrahydropyran- oder Valerolacton-System gebunden sind; |
| $M^1$, $M^2$, $M^3$, $M^4$, $M^5$, $M^6$ | sind gleich oder verschieden -O-, -CO-, -CO-O-, -O-CO-, -O-CO-O-, -O-CS-O-, $-CH_2-O-$, $-O-CH_2-$, -CH=CH-, -C≡C- oder eine Einfachbindung; |
| $A^1$, $A^2$, $A^3$, $A^4$ | sind gleich oder verschieden 1,4-Phenylen, wobei ein oder mehrere H-Atome durch F, Cl und/oder CN ersetzt sein können, Pyrazin-2,5-diyl, wobei ein oder zwei H-Atome durch F, Cl und/oder CN ersetzt sein können, Pyridazin-3,6-diyl, wobei ein oder zwei H-Atome durch F, Cl und/oder CN ersetzt sein können, Pyridin-2,5-diyl, wobei ein oder mehrere H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, wobei ein oder zwei H-Atome durch CN und/oder $CH_3$ ersetzt sein können, (1,3,4)-Thiadiazol-2,5-diyl, 1,3-Dioxan-2,5-diyl, Thiophen-2,4-diyl, wobei ein H-Atom durch F, Cl und/oder CN ersetzt sein kann,Thiophen-2,5-diyl, wobei ein oder zwei H-Atome durch F, Cl und/oder CN ersetzt sein können, Naphthalin-2,6-diyl, wobei ein oder mehrere H-Atome durch F, Cl und/oder CN ersetzt sein können, oder die Gruppe B; |
| a, b, c, d, e, f | sind null oder eins, mit der Maßgabe, daß die Summe aus b, c, d und e 0, 1 oder 2 ist. |

**3.** 1 -Fluor-6,7-dihydro-5H-isochinolin-8-on-Derivate nach Anspruch 1 und/oder 2, dadurch gekennzeichnet, daß die Symbole und Indizes in der Formel (I) folgende Bedeutungen haben:

| | |
|---|---|
| $R^1$, $R^2$ | sind gleich oder verschieden Wasserstoff, -CN, -F, -Cl, $-CF_3$, $-CHF_2$, $-CH_2F$, $-OCF_3$, $-OCHF_2$, $-OCH_2F$ oder ein geradkettiger oder verzweigter Alkylrest mit 1 bis 16 C-Atomen (mit oder ohne asymmetrisches C-Atom), wobei auch eine, zwei oder drei $CH_2$-Gruppen durch -O-, -CO-, -O-CO-, -CO-O-, -CH = CH-, Cyclopropan-1,2-diyl, $-Si(CH_3)_2-$ oder trans-1,4-Cyclohexylen ersetzt sein können, mit der Maßgabe, daß Sauerstoffatome nicht unmittelbar miteinander gebunden sein dürfen, und/oder wobei ein oder mehrere H-Atome des Alkylrestes durch -F, -Cl oder $-OR^3$ substituiert sein können, oder auch eine der nachfolgenden Gruppen (optisch aktiv oder race- |

misch):

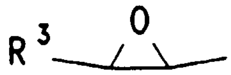
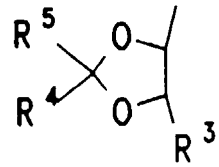
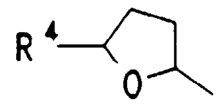

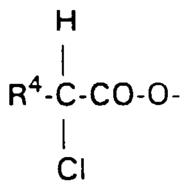
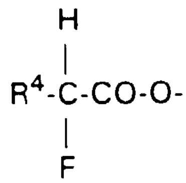
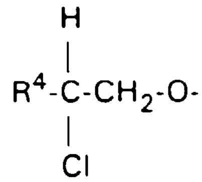
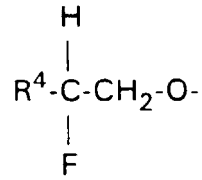

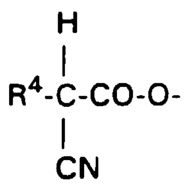
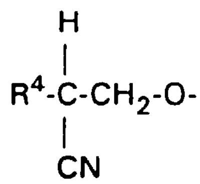
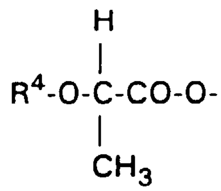
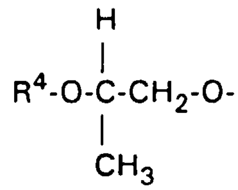

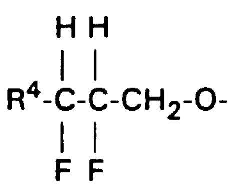
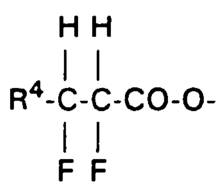

| | |
|---|---|
| $R^3$, $R^4$, $R^5$ | sind gleich oder verschieden Wasserstoff oder ein geradkettiger oder verzweigter Alkylrest mit 1-9 C-Atomen (mit oder ohne asymmetrisches C-Atom), wobei auch eine oder mehrere $CH_2$-Gruppen durch -O- und/oder -CH=CH- ersetzt sein können, mit der Maßgabe, daß Sauerstoffatome nicht unmittelbar miteinander gebunden sein dürfen, und/oder wobei ein oder mehrere H-Atome des Alkylrestes durch -F oder -Cl substituiert sein können; $R^4$ und $R^5$ können zusammen auch $-(CH_2)_4-$ oder $-(CH_2)_5-$ sein, wenn sie an ein Dioxolan-System gebunden sind; |
| $M^1$, $M^2$, $M^3$, $M^4$, $M^5$, $M^6$ | sind gleich oder verschieden -O-, -CO-, -CO-O-, -O-CO-, -O-CO-O-, $-CH_2-O-$, $-O-CH_2-$, -CH = CH- oder eine Einfachbindung; |
| $A^1$, $A^2$, $A^3$, $A^4$ | sind gleich oder verschieden 1,4-Phenylen, wobei ein, zwei oder drei H-Atome durch F, Cl und/oder CN ersetzt sein können, Pyridin-2,5-diyl, wobei ein oder zwei H-Atome durch F, Cl und/oder CN ersetzt sein können, Pyrimidin-2,5-diyl, wobei ein oder zwei H-Atome durch F, Cl und/oder CN ersetzt sein können, trans-1,4-Cyclohexylen, wobei ein oder zwei H-Atome durch CN und/oder $CH_3$ ersetzt sein können, (1,3,4)-Thiadiazol-2,5-diyl oder Naphthalin-2,6-diyl, wobei ein oder zwei H-Atome durch F, Cl und/oder CN ersetzt sein können; |
| a, b, c, d, e, f | sind null oder eins, mit der Maßgabe, daß die Summe aus b, c, d und e 0, 1 oder 2 ist. |

4. 1-Fluor-6,7-dihydro-5H-isochinolin-8-on-Derivate nach einem oder mehreren der Ansprüche 1 bis 3, dadurch gekennzeichnet, daß die Gruppe $(-A^1-M^2)_b(-A^2-M^3)_c-B-(M^4-A^3)_d(-M^5-A^4)_e$ eine der folgenden Bedeutungen hat:

| | | |
|---|---|---|
| -Phe-Phe-(F)ICH | -Phe-Pym-(F)ICH | -Phe-Pyr-(F)ICH |
| -Phe-Diox-(F)ICH | -Phe-Naf-(F)ICH | -Phe-$F_2$Phe-(F)ICH |
| -Phe-(F)Pyr-(F)ICH | -Phe-(F)Phe-(F)ICH | -Phe-TDZ-(F)ICH |
| -Pym-Phe-(F)ICH | -Pym-Pym-(F)ICH | -Pym-Pyr-(F)ICH |
| -Pym-Diox-(F)ICH | -Pym-Naf-(F)ICH | -Pym-$F_2$Phe-(F)ICH |
| -Pym-(F)Pyr-(F)ICH | -Pym-(F)Phe-(F)ICH | -Pym-TDZ-(F)ICH |
| -Pyr-Phe-(F)ICH | -Pyr-Pym-(F)ICH | -Pyr-Pyr-(F)ICH |
| -Pyr-Diox-(F)ICH | -Pyr-Naf-(F)ICH | -Pyr-$F_2$Phe-(F)ICH |
| -Pyr-(F)Pyr-(F)ICH | -Pyr-(F)Phe-(F)ICH | -Pyr-TDZ-(F)ICH |
| -Diox-Phe-(F)ICH | -Diox-Pym-(F)ICH | -Diox-Pyr-(F)ICH |
| -Diox-Diox-(F)ICH | -Diox-Naf-(F)ICH | -Diox-$F_2$Phe-(F)ICH |
| -Diox-(F)Pyr-(F)ICH | -Diox-(F)Phe-(F)ICH | -Diox-TDZ-(F)ICH |
| -Naf-Phe-(F)ICH | -Naf-Pym-(F)ICH | -Naf-Pyr-(F)ICH |
| -Naf-Diox-(F)ICH | -Naf-Naf-(F)ICH | -Naf-$F_2$Phe-(F)ICH |
| -Naf-(F)Pyr-(F)ICH | -Naf-(F)Phe-(F)ICH | -Naf-TDZ-(F)ICH |
| -$F_2$Phe-Phe-(F)ICH | -$F_2$Phe-Pym-(F)ICH | -$F_2$Phe-Pyr-(F)ICH |
| -$F_2$Phe-Diox-(F)ICH | -$F_2$Phe-Naf-(F)ICH | -$F_2$Phe-$F_2$Phe-(F)ICH |
| -$F_2$Phe-(F)Pyr-(F)ICH | -$F_2$Phe-(F)Phe-(F)ICH | -$F_2$Phe-TDZ-(F)ICH |
| -(F)Pyr-Phe-(F)ICH | -(F)Pyr-Pym-(F)ICH | -(F)Pyr-Pyr-(F)ICH |
| -(F)Pyr-Diox-(F)ICH | -(F)Pyr-Naf-(F)ICH | -(F)Pyr-$F_2$Phe-(F)ICH |
| -(F)Pyr-(F)Pyr-(F)ICH | -(F)Pyr-(F)Phe-(F)ICH | -(F)Pyr-TDZ-(F)ICH |
| -(F)Phe-Phe-(F)ICH | -(F)Phe-Pym-(F)ICH | -(F)Phe-Pyr-(F)ICH |
| -(F)Phe-Diox-(F)ICH | -(F)Phe-Naf-(F)ICH | -(F)Phe-$F_2$Phe-(F)ICH |
| -(F)Phe-(F)Pyr-(F)ICH | -(F)Phe-(F)Phe-(F)ICH | -(F)Phe-TDZ-(F)ICH |
| -TDZ-Phe-(F)ICH | -TDZ-Pym-(F)ICH | -TDZ-Pyr-(F)ICH |
| -TDZ-Diox-(F)ICH | -TDZ-Naf-(F)ICH | -TDZ-$F_2$Phe-(F)ICH |
| -TDZ-(F)Pyr-(F)ICH | -TDZ-(F)Phe-(F)ICH | -TDZ-TDZ-(F)ICH |
| -Phe-(F)ICH | -Pym-(F)ICH | -Pyr-(F)ICH |
| -Diox-(F)ICH | -Naf-(F)ICH | -$F_2$Phe-(F)ICH |
| -(F)Pyr-(F)ICH | -(F)Phe-(F)ICH | -TDZ-(F)ICH |
| -(F)ICH-Phe-Phe | -(F)ICH-Pym-Phe | -(F)ICH-Pyr-Phe |
| -(F)ICH-Diox-Phe | -(F)ICH-Naf-Phe | -(F)ICH-$F_2$Phe-Phe |
| -(F)ICH-(F)Pyr-Phe | -(F)ICH-(F)Phe-Phe | -(F)ICH-TDZ-Phe |
| -(F)ICH-Phe-Pym | -(F)ICH-Pym-Pym | -(F)ICH-Pyr-Pym |
| -(F)ICH-Diox-Pym | -(F)ICH-Naf-Pym | -(F)ICH-$F_2$Phe-Pym |
| -(F)ICH-(F)Pyr-Pym | -(F)ICH-(F)Phe-Pym | -(F)ICH-TDZ-Pym |
| -(F)ICH-Phe-Pyr | -(F)ICH-Pym-Pyr | -(F)ICH-Pyr-Pyr |
| -(F)ICH-Diox-Pyr | -(F)ICH-Naf-Pyr | -(F)ICH-$F_2$Phe-Pyr |
| -(F)ICH-(F)Pyr-Pyr | -(F)ICH-(F)Phe-Pyr | -(F)ICH-TDZ-Pyr |
| -(F)ICH-Phe-Diox | -(F)ICH-Pym-Diox | -(F)ICH-Pyr-Diox |
| -(F)ICH-Diox-Diox | -(F)ICH-Naf-Diox | -(F)ICH-$F_2$Phe-Diox |
| -(F)ICH-(F)Pyr-Diox | -(F)ICH-(F)Phe-Diox | -(F)ICH-TDZ-Diox |
| -(F)ICH-Phe-Naf | -(F)ICH-Pym-Naf | -(F)ICH-Pyr-Naf |
| -(F)ICH-Diox-Naf | -(F)ICH-Naf-Naf | -(F)ICH-$F_2$Phe-Naf |
| -(F)ICH-(F)Pyr-Naf | -(F)ICH-(F)Phe-Naf | -(F)ICH-TDZ-Naf |
| -(F)ICH-Phe-$F_2$Phe | -(F)ICH-Pym-$F_2$Phe | -(F)ICH-Pyr-$F_2$Phe |
| -(F)ICH-Diox-$F_2$Phe | -(F)ICH-Naf-$F_2$Phe | -(F)ICH-$F_2$Phe-$F_2$Phe |
| -(F)ICH-(F)Pyr-$F_2$Phe | -(F)ICH-(F)Phe-$F_2$Phe | -(F)ICH-TDZ-$F_2$Phe |
| -(F)ICH-Phe-(F)Pyr | -(F)ICH-Pym-(F)Pyr | -(F)ICH-Pyr-(F)Pyr |

(fortgesetzt)

| | | |
|---|---|---|
| -(F)ICH-Diox-(F)Pyr | -(F)ICH-Naf-(F)Pyr | -(F)ICH-$F_2$Phe-(F)Pyr |
| -(F)ICH-(F)Pyr-(F)Pyr | -(F)ICH-(F)Phe-(F)Pyr | -(F)ICH-TDZ-(F)Pyr |
| -(F)ICH-Phe-(F)Phe | -(F)ICH-Pym-(F)Phe | -(F)ICH-Pyr-(F)Phe |
| -(F)ICH-Diox-(F)Phe | -(F)ICH-Naf-(F)Phe | -(F)ICH-$F_2$Phe-(F)Phe |
| -(F)ICH-(F)Pyr-(F)Phe | -(F)ICH-(F)Phe-(F)Phe | -(F)ICH-TDZ-(F)Phe |
| -(F)ICH-Phe-TDZ | -(F)ICH-Pym-TDZ | -(F)ICH-Pyr-TDZ |
| -(F)ICH-Diox-TDZ | -(F)ICH-Naf-TDZ | -(F)ICH-$F_2$Phe-TDZ |
| -(F)ICH-(F)Pyr-TDZ | -(F)ICH-(F)Phe-TDZ | -(F)ICH-TDZ-TDZ |
| -(F)ICH-Phe | -(F)ICH-Pym | -(F)ICH-Pyr |
| -(F)ICH-Diox | -(F)ICH-Naf | -(F)ICH-$F_2$Phe |
| -(F)ICH-(F)Pyr | -(F)ICH-(F)Phe | -(F)ICH-TDZ |
| -Phe-(F)ICH-Phe | -Pym-(F)ICH-Phe | -Pyr-(F)ICH-Phe |
| -Diox-(F)ICH-Phe | -Naf-(F)ICH-Phe | -$F_2$Phe-(F)ICH-Phe |
| -(F)Pyr-(F)ICH-Phe | -(F)Phe-(F)ICH-Phe | -TDZ-(F)ICH-Phe |
| -Phe-(F)ICH-Pym | -Pym-(F)ICH-Pym | -Pyr-(F)ICH-Pym |
| -Diox-(F)ICH-Pym | -Naf-(F)ICH-Pym | -$F_2$Phe-(F)ICH-Pym |
| -(F)Pyr-(F)ICH-Pym | -(F)Phe-(F)ICH-Pym | -TDZ-(F)ICH-Pym |
| -Phe-(F)ICH-Pyr | -Pym-(F)ICH-Pyr | -Pyr-(F)ICH-Pyr |
| -Diox-(F)ICH-Pyr | -Naf-(F)ICH-Pyr | -$F_2$Phe-(F)ICH-Pyr |
| -(F)Pyr-(F)ICH-Pyr | -(F)Phe-(F)ICH-Pyr | -TDZ-(F)ICH-Pyr |
| -Phe-(F)ICH-Diox | -Pym-(F)ICH-Diox | -Pyr-(F)ICH-Diox |
| -Diox-(F)ICH-Diox | -Naf-(F)ICH-Diox | -$F_2$Phe-(F)ICH-Diox |
| -(F)Pyr-(F)ICH-Diox | -(F)Phe-(F)ICH-Diox | -TDZ-(F)ICH-Diox |
| -Phe-(F)ICH-Naf | -Pym-(F)ICH-Naf | -Pyr-(F)ICH-Naf |
| -Diox-(F)ICH-Naf | -Naf-(F)ICH-Naf | -$F_2$Phe-(F)ICH-Naf |
| -(F)Pyr-(F)ICH-Naf | -(F)Phe-(F)ICH-Naf | -TDZ-(F)ICH-Naf |
| -Phe-(F)ICH-$F_2$Phe | -Pym-(F)ICH-$F_2$Phe | -Pyr-(F)ICH-$F_2$Phe |
| -Diox-(F)ICH-$F_2$Phe | -Naf-(F)ICH-$F_2$Phe | -$F_2$Phe-(F)ICH-$F_2$Phe |
| -(F)Pyr-(F)ICH-$F_2$Phe | -(F)Phe-(F)ICH-$F_2$Phe | -TDZ-(F)ICH-$F_2$Phe |
| -Phe-(F)ICH-(F)Pyr | -Pym-(F)ICH-(F)Pyr | -Pyr-(F)ICH-(F)Pyr |
| -Diox-(F)ICH-(F)Pyr | -Naf-(F)ICH-(F)Pyr | -$F_2$Phe-(F)ICH-(F)Pyr |
| -(F)Pyr-(F)ICH-(F)Pyr | -(F)Phe-(F)ICH-(F)Pyr | -TDZ-(F)ICH-(F)Pyr |
| -Phe-(F)ICH-(F)Phe | -Pym-(F)ICH-(F)Phe | -Pyr-(F)ICH-(F)Phe |
| -Diox-(F)ICH-(F)Phe | -Naf-(F)ICH-(F)Phe | -$F_2$Phe-(F)ICH-(F)Phe |
| -(F)Pyr-(F)ICH-(F)Phe | -(F)Phe-(F)ICH-(F)Phe | -TDZ-(F)ICH-(F)Phe |
| -Phe-(F)ICH-TDZ | -Pym-(F)ICH-TDZ | -Pyr-(F)ICH-TDZ |
| -Diox-(F)ICH-TDZ | -Naf-(F)ICH-TDZ | -$F_2$Phe-(F)ICH-TDZ |
| -(F)Pyr-(F)ICH-TDZ | -(F)Phe-(F)ICH-TDZ | -TDZ-(F)ICH-TDZ |

wobei die Abkürzungen

| | |
|---|---|
| (F)ICH | 1-Fluor-6,7-dihydro-5H-isochinolin-8-on-3,7-diyl, |
| Phe | 1,4-Phenylen, |
| Pyr | Pyridin-2,5-diyl, |
| Pym | Pyrimidin-2,5-diyl, |
| Diox | 1,3-Dioxan-2,5-diyl, |
| Naf | Naphthalin-2,6-diyl, |
| (F)Phe | Fluorbenzol-1,4-diyl, |
| $F_2$Phe | Difluorbenzol-1,4-diyl, |
| (F)Pyr | Fluorpyridin-2,5-diyl und |
| TDZ | (1,3,4)-Thiadiazol-2,5-diyl |

bedeuten und $M^1$, $M^6$, $R^1$, $R^2$ die in der Formel (I) angegebenen Bedeutungen haben.

**5.** Verwendung von 1 -Fluor-6,7-dihydro-5H-isochinolin-8-on-Derivate der Formel (I) nach einem oder mehreren der Ansprüche 1 bis 4 als Komponenten flüssigkristalliner Mischungen.

**6.** Flüssigkristallmischung, enthaltend eine oder mehrere Verbindungen der Formel (I) nach einem oder mehreren der Ansprüche 1 bis 4.

**7.** Flüssigkristallmischung nach Anspruch 6, dadurch gekennzeichnet, daß sie ferroelektrisch ist.

**8.** Flüssigkristallmischung nach Anspruch 6 oder 7, dadurch gekennzeichnet, daß sie 0, 1 bis 60 Mol-% einer oder mehrerer Verbindungen der Formel (I) enthält.

**9.** Flüssigkristallmischung nach einem oder mehreren der Ansprüche 6 bis 8, dadurch gekennzeichnet, daß sie 1 bis 10 Verbindungen der Formel (I) enthält.

**10.** Schalt- und/oder Anzeigevorrichtung, enthaltend Trägerplatten, Elektroden, mindestens einen Polarisator, mindestens eine Orientierungsschicht sowie ein flüssigkristallines Medium, dadurch gekennzeichnet, daß das flüssigkristalline Medium eine Flüssigkristallmischung nach einem oder mehreren der Ansprüche 6 bis 9 ist.

## Claims

**1.** A 1-fluoro-6,7-dihydro-5H-isoquinolin-8-one derivative of the formula (I)

$$R^1(-M^1)_a(-A^1-M^2)_b(-A^2-M^3)_c-B(-M^4-A^3)_d(-M^5-A^4)_e(-M^6)_f-R^2 \qquad (I)$$

in which the symbols and indices have the following meanings: the group B is

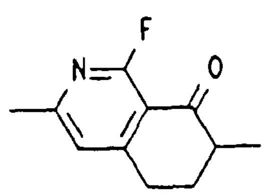

$R^1$ and $R^2$ are identical or different and are hydrogen, -CN, -F, -Cl, $-CF_3$, $-CHF_2$, $-CH_2F$, $-OCF_3$, $-OCHF_2$, $-OCH_2F$ or a straight-chain or branched alkyl radical having 1 to 20 carbon atoms (with or without an asymmetrical carbon atom), where one or more $CH_2$ groups may also be replaced by -O-, -S-, -CO-O-, -O-CO-, -O-CO-O-, -CO-, -CS-, -CH=CH-, -C≡C-, cyclopropane-1,2-diyl, $-Si(CH_3)_2-$, 1,4-phenylene, trans-1,4-cyclohexylene or trans-1,3-cyclopentylene, with the proviso that oxygen atoms and/or sulfur atoms must not be bonded directly to one another, and/or where one or more H atoms of the alkyl radical may be substituted by -F, -Cl, -Br, $-OR^3$, -SCN, -OCN or $-N_3$ or are alternatively one of the following groups (optically active or racemic):

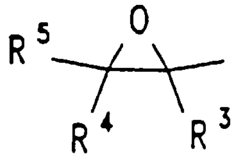

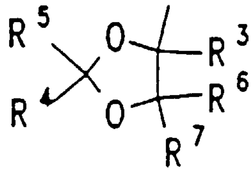

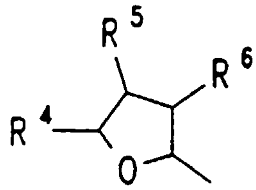

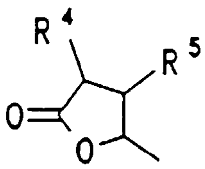

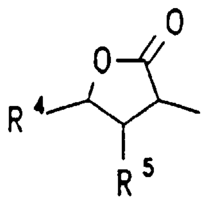

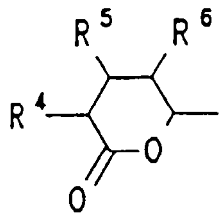

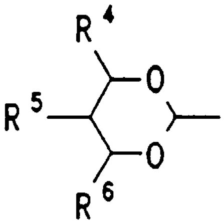

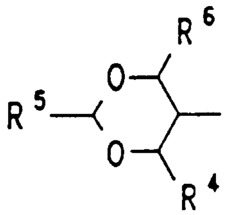

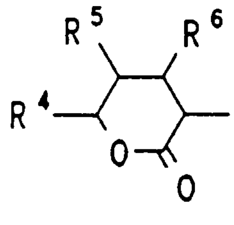

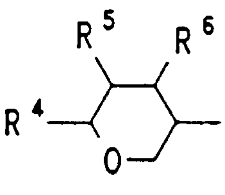

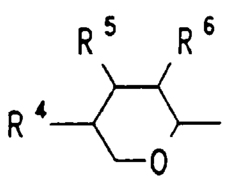

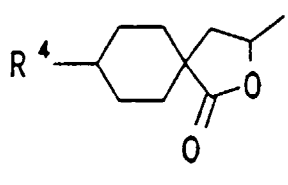

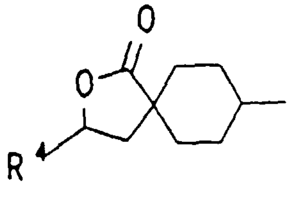

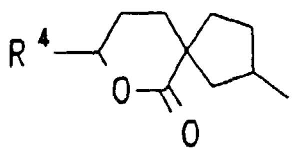

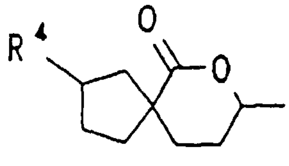

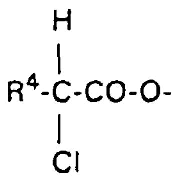 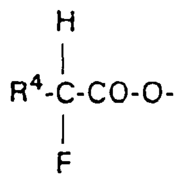 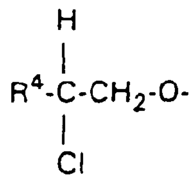 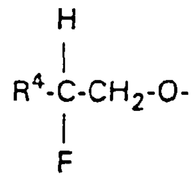

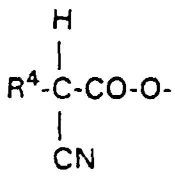 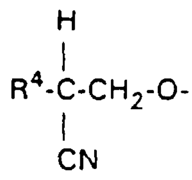 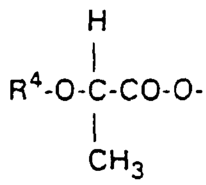 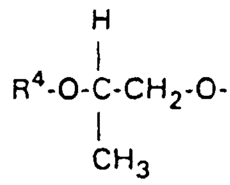

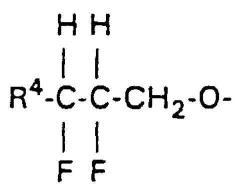 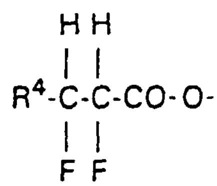

| | |
|---|---|
| $R^3$, $R^4$, $R^5$, $R^6$ and $R^7$ | are identical or different and are hydrogen or a straight-chain or branched alkyl radical having 1-16 carbon atoms (with or without an asymmetrical carbon atom), where one or more $CH_2$ groups may also be replaced by -O- and/or -CH=CH-, with the proviso that oxygen atoms must not be bonded directly to one another, and/or where one or more H atoms of the alkyl radical may be substituted by -F or -Cl; $R^4$ and $R^5$ may also together be $-(CH_2)_4-$ or $-(CH_2)_5-$ if they are bonded to an oxirane, dioxolane, tetrahydrofuran, tetrahydropyran, butyrolactone or valerolactone system; |
| $M^1$, $M^2$, $M^3$, $M^4$, $M^5$ and $M^6$ | are identical or different and are -O-, -S-, -CO-, -CO-O-, -O-CO-, -O-CO-O-, -CO-S-, -S-CO-, -CS-O-, -O-CS-, -S-CS-S-, -O-CS-O-, -S-CO-S-, -CS-, $-CH_2-O-$, $-O-CH_2-$, $-CH_2-S-$, $-S-CH_2-$, -CH=CH-, -C≡C-, $-CH_2-CH_2-CO-O$, $-O-CO-CH_2-CH_2-$ or a single bond; |
| $A^1$, $A^2$, $A^3$ and $A^4$ | are identical or different and are 1,4-phenylene, in which one or more H atoms may be replaced by F, Cl and/or CN, pyrazine-2,5-diyl, in which one or two H atoms may be replaced by F, Cl and/or CN, pyridazine-3,6-diyl, in which one or two H atoms may be replaced by F, Cl and/or CN, pyridine-2,5-diyl, in which one or more H atoms may be replaced by F, Cl and/or CN, pyrimidine-2,5-diyl, in which one or two H atoms may be replaced by F, Cl and/or CN, trans-1,4-cyclohexylene, in which one or two H atoms may be replaced by CN and/or $CH_3$, 1,3,4-thiadiazole-2,5-diyl, 1,3-dioxane-2,5-diyl, 1,3-dithiane-2,5-diyl, 1,3-thiazole-2,4-diyl, in which one H atom may be replaced by F, Cl and/ or CN, 1,3-thiazole-2,5-diyl, in which one H atom may be replaced by F, Cl and/ or CN, thiophene-2,4-diyl, in which one H atom may be replaced by F, Cl and/or CN, thiophene-2,5-diyl, in which one or two H atoms may be replaced by F, Cl and/or CN, piperazine-1,4-diyl, piperazine-2,5-diyl, naphthalene-2,6-diyl, in which one or more H atoms may be replaced by F, Cl and/or CN, bicyclo[2.2.2]octane-1,4-diyl, in which one or more H atoms may be replaced by F, Cl and/or CN, 1,3-dioxaborinane-2,5-diyl or the group B; |
| a, b, c, d, e and f | are zero or one, with the proviso that the sum of b, c, d and e must be 0, 1 or 2. |

**2.** A 1-fluoro-6,7-dihydro-5H-isoquinolin-8-one derivative as claimed in claim 1, wherein the symbols and indices in the formula (I) have the following meanings:

$R^1$ and $R^2$ are identical or different and are hydrogen, -CN, -F, -Cl, $-CF_3$, $-CHF_2$, $-CH_2F$, $-OCF_3$, $-OCHF_2$, $-OCH_2F$ or a straight-chain or branched alkyl radical having 1 to 18 carbon atoms (with or without an asymmetrical carbon atom), where one or more $-CH_2-$ groups may also be replaced by -O-, -CO-, -CO-O-, -O-CO-, -O-CO-O-, -CH=CH-, -C≡C-, cyclopropane-1,2-diyl, $-Si(CH_3)_2-$ or trans-1,4-cyclohexylene, with the proviso that oxygen atoms must not be bonded directly to one another, and/or where one or more H atoms of the alkyl radical may be substituted by -F, -Cl, $-OR^3$, -OCN or $-N_3$, or are alternatively one of the following groups (optically active or racemic):

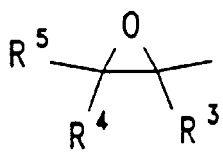
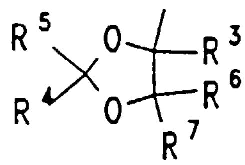
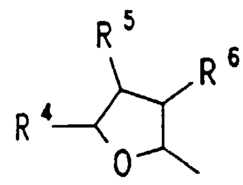

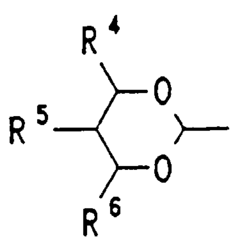
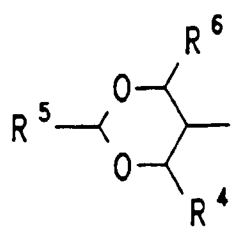
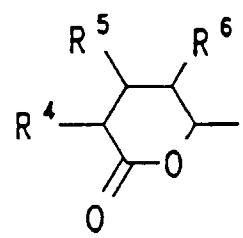

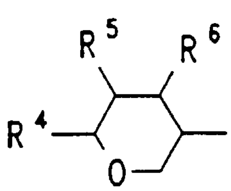
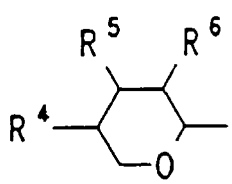
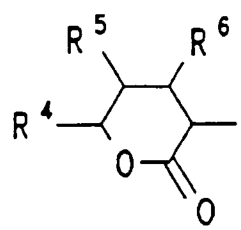

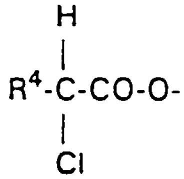
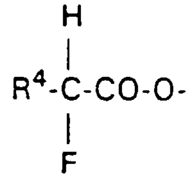
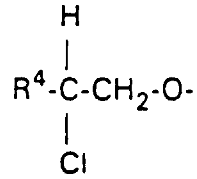
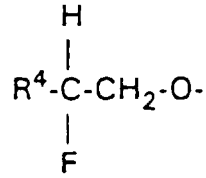

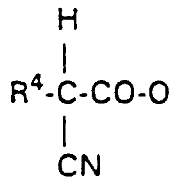
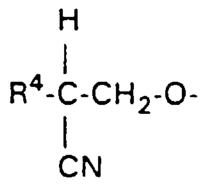
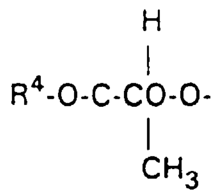
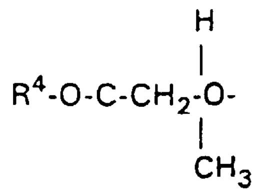

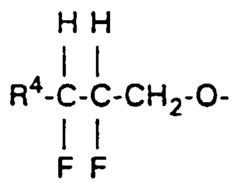
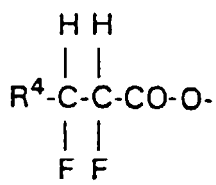

| | |
|---|---|
| $R^3$, $R^4$, $R^5$, $R^6$ and $R^7$ | are identical or different and are hydrogen or a straight-chain or branched alkyl radical having 1-16 carbon atoms (with or without an asymmetrical carbon atom), where one or more $CH_2$ groups may also be replaced by -O- and/or -CH=CH-, with the proviso that oxygen atoms must not be bonded directly to one another, and/or where one or more H atoms of the alkyl radical may be substituted by -F or -Cl; $R^4$ and $R^5$ may also together be $-(CH_2)_4-$ or $-(CH_2)_5-$ if they are bonded to an oxirane, dioxolane, tetrahydrofuran, tetrahydropyran or valerolactone system; |
| $M^1$, $M^2$, $M^3$, $M^4$, $M^5$ and $M^6$ | are identical or different and are -O-, -CO-, -CO-O-, -O-CO-, -O-CO-O-, -O-CS-O-, $-CH_2-O-$, $-O-CH_2-$, -CH=CH-, -C≡C- or a single bond; |
| $A^1$, $A^2$, $A^3$ and $A^4$ | are identical or different and are 1,4-phenylene, in which one or more H atoms may be replaced by F, Cl and/or CN, pyrazine-2,5-diyl, in which one or two H atoms may be replaced by F, Cl and/or CN, pyridazine-3,6-diyl, in which one or two H atoms may be replaced by F, Cl and/or CN, pyridine-2,5-diyl, in which one or more H atoms may be replaced by F, Cl and/or CN, pyrimidine-2,5-diyl, in which one or two H atoms may be replaced by F, Cl and/or CN, trans-1,4-cyclohexylene, in which one or two H atoms may be replaced by CN and/or $CH_3$, 1,3,4-thiadiazole-2,5-diyl, 1,3-dioxane-2,5-diyl, thiophene-2,4-diyl, in which one H atom may be replaced by F, Cl and/or CN, thiophene-2,5-diyl, in which one or two H atoms may be replaced by F, Cl and/or CN, naphthalene-2,6-diyl, in which one or more H atoms may be replaced by F, Cl and/or CN, or the group B; |
| a, b, c, d, e and f | are zero or one, with the proviso that the sum of b, c, d and e is 0, 1 or 2. |

**3.** A 1-fluoro-6,7-dihydro-5H-isoquinolin-8-one derivative as claimed in claim 1 and/or 2, wherein the symbols and indices in the formula (I) have the following meanings:

| | |
|---|---|
| $R^1$ and $R^2$ | are identical or different and are hydrogen, -CN, -F, -Cl, $-CF_3$, $-CHF_2$, $-CH_2F$, $-OCF_3$, $-OCHF_2$, $-OCH_2F$ or a straight-chain or branched alkyl radical having 1 to 16 carbon atoms (with or without an asymmetrical carbon atom), where one, two or three $-CH_2-$ groups may also be replaced by -O-, -CO-, -O-CO-, -CO-O-, -CH=CH-, cyclopropane-1,2-diyl, $-Si(CH_3)_2-$ or trans-1,4-cyclohexylene, with the proviso that oxygen atoms must not be bonded directly to one another, and/or where one or more H atoms of the alkyl radical may be substituted by -F, -Cl or $-OR^3$, or are alternatively one of the following groups (optically active or racemic): |

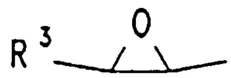 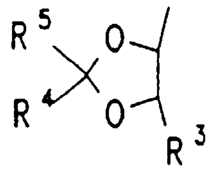 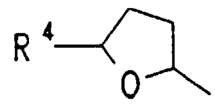

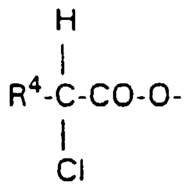 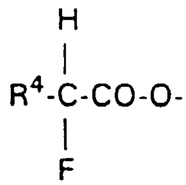 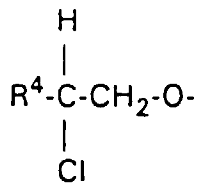 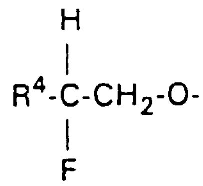

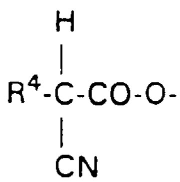 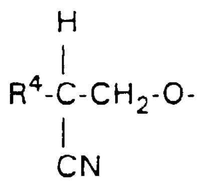 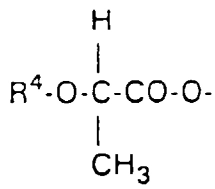 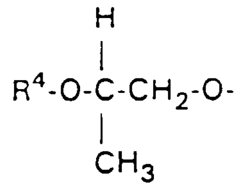

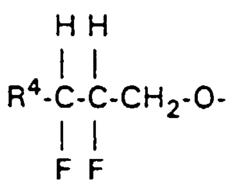 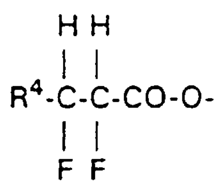

| | |
|---|---|
| $R^3$, $R^4$ and $R^5$ | are identical or different and are hydrogen or a straight-chain or branched alkyl radical having 1-9 carbon atoms (with or without an asymmetrical carbon atom), where one or more $CH_2$ groups may also be replaced by -O- and/or -CH=CH-, with the proviso that oxygen atoms must not be bonded directly to one another, and/or where one or more H atoms of the alkyl radical may be substituted by -F or -Cl; $R^4$ and $R^5$ may also together be $-(CH_2)_4-$ or $-(CH_2)_5-$ if they are bonded to a dioxolane system; |
| $M^1$, $M^2$, $M^3$, $M^4$, $M^5$ and $M^6$ | are identical or different and are -O-, -CO-, -CO-O-, -O-CO-, -O-CO-O-, $-CH_2-O-$, $-O-CH_2-$, -CH=CH- or a single bond; |
| $A^1$, $A^2$, $A^3$ and $A^4$ | are identical or different and are 1,4-phenylene, in which one, two or three H atoms may be replaced by F, Cl and/or CN, pyridine-2,5-diyl, in which one or two H atoms may be replaced by F, Cl and/or CN, pyrimidine-2,5-diyl, in which one or two H atoms may be replaced by F, Cl and/ or CN, trans-1,4-cyclohexylene, in which one or two H atoms may be replaced by CN and/or $CH_3$, 1,3,4-thiadiazole-2,5-diyl or naphthalene-2,6-diyl, in which one or two H atoms may be replaced by F, Cl and/or CN; |
| a, b, c, d, e and f | are zero or one, with the proviso that the sum of b, c, d and e is 0, 1 or 2. |

**4.** A 1-fluoro-6,7-dihydro-5H-isoquinolin-8-one derivative as claimed in one or more of claims 1 to 3, wherein the $(-A^1-M^2)_b(-A^2-M^3)_c-B-(M^4-A^3)_d(-M^5-A^4)_e$ group has one of the following meanings:

| | | |
|---|---|---|
| -Phe-Phe-(F)ICH | -Phe-Pym-(F)ICH | -Phe-Pyr-(F)ICH |
| -Phe-Diox-(F)ICH | -Phe-Naf-(F)ICH | -Phe-$F_2$Phe-(F)ICH |
| -Phe-(F)Pyr-(F)ICH | -Phe-(F)Phe-(F)ICH | -Phe-TDZ-(F)ICH |
| -Pym-Phe-(F)ICH | -Pym-Pym-(F)ICH | -Pym-Pyr-(F)ICH |
| -Pym-Diox-(F)ICH | -Pym-Naf-(F)ICH | -Pym-$F_2$Phe-(F)ICH |
| -Pym-(F)Pyr-(F)ICH | -Pym-(F)Phe-(F)ICH | -Pym-TDZ-(F)ICH |
| -Pyr-Phe-(F)ICH | -Pyr-Pym-(F)ICH | -Pyr-Pyr-(F)ICH |
| -Pyr-Diox-(F)ICH | -Pyr-Naf-(F)ICH | -Pyr-$F_2$Phe-(F)ICH |
| -Pyr-(F)Pyr-(F)ICH | -Pyr-(F)Phe-(F)ICH | -Pyr-TDZ-(F)ICH |
| -Diox-Phe-(F)ICH | -Diox-Pym-(F)ICH | -Diox-Pyr-(F)ICH |
| -Diox-Diox-(F)ICH | -Diox-Naf-(F)ICH | -Diox-$F_2$Phe-(F)ICH |
| -Diox-(F)Pyr-(F)ICH | -Diox-(F)Phe-(F)ICH | -Diox-TDZ-(F)ICH |
| -Naf-Phe-(F)ICH | -Naf-Pym-(F)ICH | -Naf-Pyr-(F)ICH |
| -Naf-Diox-(F)ICH | -Naf-Naf-(F)ICH | -Naf-$F_2$Phe-(F)ICH |
| -Naf-(F)Pyr-(F)ICH | -Naf-(F)Phe-(F)ICH | -Naf-TDZ-(F)ICH |
| -$F_2$Phe-Phe-(F)ICH | -$F_2$Phe-Pym-(F)ICH | -$F_2$Phe-Pyr-(F)ICH |
| -$F_2$Phe-Diox-(F)ICH | -$F_2$Phe-Naf-(F)ICH | -$F_2$Phe-$F_2$Phe-(F)ICH |
| -$F_2$Phe-(F)Pyr-(F)ICH | -$F_2$Phe-(F)Phe-(F)ICH | -$F_2$Phe-TDZ-(F)ICH |
| -(F)Pyr-Phe-(F)ICH | -(F)Pyr-Pym-(F)ICH | -(F)Pyr-Pyr-(F)ICH |
| -(F)Pyr-Diox-(F)ICH | -(F)Pyr-Naf-(F)ICH | -(F)Pyr-$F_2$Phe-(F)ICH |
| -(F)Pyr-(F)Pyr-(F)ICH | -(F)Pyr-(F)Phe-(F)ICH | -(F)Pyr-TDZ-(F)ICH |
| -(F)Phe-Phe-(F)ICH | -(F)Phe-Pym-(F)ICH | -(F)Phe-Pyr-(F)ICH |
| -(F)Phe-Diox-(F)ICH | -(F)Phe-Naf-(F)ICH | -(F)Phe-$F_2$Phe-(F)ICH |
| -(F)Phe-(F)Pyr-(F)ICH | -(F)Phe-(F)Phe-(F)ICH | -(F)Phe-TDZ-(F)ICH |
| -TDZ-Phe-(F)ICH | -TDZ-Pym-(F)ICH | -TDZ-Pyr-(F)ICH |
| -TDZ-Diox-(F)ICH | -TDZ-Naf-(F)ICH | -TDZ-$F_2$Phe-(F)ICH |
| -TDZ-(F)Pyr-(F)ICH | -TDZ-(F)Phe-(F)ICH | -TDZ-TDZ-(F)ICH |
| -Phe-(F)ICH | -Pym-(F)ICH | -Pyr-(F)ICH |
| -Diox-(F)ICH | -Naf-(F)ICH | -$F_2$Phe-(F)ICH |
| -(F)Pyr-(F)ICH | -(F)Phe-(F)ICH | -TDZ-(F)ICH |
| -(F)ICH-Phe-Phe | -(F)ICH-Pym-Phe | -(F)ICH-Pyr-Phe |
| -(F)ICH-Diox-Phe | -(F)ICH-Naf-Phe | -(F)ICH-$F_2$Phe-Phe |
| -(F)ICH-(F)Pyr-Phe | -(F)ICH-(F)Phe-Phe | -(F)ICH-TDZ-Phe |
| -(F)ICH-Phe-Pym | -(F)ICH-Pym-Pym | -(F)ICH-Pyr-Pym |
| -(F)ICH-Diox-Pym | -(F)ICH-Naf-Pym | -(F)ICH-$F_2$Phe-Pym |
| -(F)ICH-(F)Pyr-Pym | -(F)ICH-(F)Phe-Pym | -(F)ICH-TDZ-Pym |
| -(F)ICH-Phe-Pyr | -(F)ICH-Pym-Pyr | -(F)ICH-Pyr-Pyr |
| -(F)ICH-Diox-Pyr | -(F)ICH-Naf-Pyr | -(F)ICH-$F_2$Phe-Pyr |
| -(F)ICH-(F)Pyr-Pyr | -(F)ICH-(F)Phe-Pyr | -(F)ICH-TDZ-Pyr |
| -(F)ICH-Phe-Diox | -(F)ICH-Pym-Diox | -(F)ICH-Pyr-Diox |
| -(F)ICH-Diox-Diox | -(F)ICH-Naf-Diox | -(F)ICH-$F_2$Phe-Diox |
| -(F)ICH-(F)Pyr-Diox | -(F)ICH-(F)Phe-Diox | -(F)ICH-TDZ-Diox |
| -(F)ICH-Phe-Naf | -(F)ICH-Pym-Naf | -(F)ICH-Pyr-Naf |
| -(F)ICH-Diox-Naf | -(F)ICH-Naf-Naf | -(P)ICH-$F_2$Phe-Naf |
| -(F)ICH-(F)Pyr-Naf | -(F)ICH-(F)Phe-Naf | -(F)ICH-TDZ-Naf |
| -(F)ICH-Phe-$F_2$Phe | -(F)ICH-Pym-$F_2$Phe | -(F)ICH-Pyr-$F_2$Phe |
| -(F)ICH-Diox-$F_2$Phe | -(F)ICH-Naf-$F_2$Phe | -(F)ICH-$F_2$Phe-$F_2$Phe |
| -(F)ICH-(F)Pyr-$F_2$Phe | -(F)ICH-(F)Phe-$F_2$Phe | -(F)ICH-TDZ-$F_2$Phe |
| -(F)ICH-Phe-(F)Pyr | -(F)ICH-Pym-(F)Pyr | -(F)ICH-Pyr-(F)Pyr |
| -(F)ICH-Diox-(F)Pyr | -(F)ICH-Naf-(F)Pyr | -(F)ICH-$F_2$Phe-(F)Pyr |
| -(F)ICH-(F)Pyr-(F)Pyr | -(F)ICH-(F)Phe-(F)Pyr | -(F)ICH-TDZ-(F)Pyr |

(continued)

| | | |
|---|---|---|
| -(F)ICH-Phe-(F)Phe | -(F)ICH-Pym-(F)Phe | -(F)ICH-Pyr-(F)Phe |
| -(F)ICH-Diox-(F)Phe | -(F)ICH-Naf-(F)Phe | -(F)ICH-$F_2$Phe-(F)Phe |
| -(F)ICH-(F)Pyr-(F)Phe | -(F)ICH-(F)Phe-(F)Phe | -(F)ICH-TDZ-(F)Phe |
| -(F)ICH-Phe-TDZ | -(F)ICH-Pym-TDZ | -(F)ICH-Pyr-TDZ |
| -(F)ICH-Diox-TDZ | -(F)ICH-Naf-TDZ | -(F)ICH-$F_2$Phe-TDZ |
| -(F)ICH-(F)Pyr-TDZ | -(F)ICH-(F)Phe-TDZ | -(F)ICH-TDZ-TDZ |
| -(F)ICH-Phe | -(F)ICH-Pym | -(F)ICH-Pyr |
| -(F)ICH-Diox | -(F)ICH-Naf | -(F)ICH-$F_2$Phe |
| -(F)ICH-(F)Pyr | -(F)ICH-(F)Phe | -(F)ICH-TDZ |
| -Phe-(F)ICH-Phe | -Pym-(F)ICH-Phe | -Pyr-(F)ICH-Phe |
| -Diox-(F)ICH-Phe | -Naf-(F)ICH-Phe | -$F_2$Phe-(F)ICH-Phe |
| -(F)Pyr-(F)ICH-Phe | -(F)Phe-(F)ICH-Phe | -TDZ-(F)ICH-Phe |
| -Phe-(F)ICH-Pym | -Pym-(F)ICH-Pym | -Pyr-(F)ICH-Pym |
| -Diox-(F)ICH-Pym | -Naf-(F)ICH-Pym | -$F_2$Phe-(F)ICH-Pym |
| -(F)Pyr-(F)ICH-Pym | -(F)Phe-(F)ICH-Pym | -TDZ-(F)ICH-Pym |
| -Phe-(F)ICH-Pyr | -Pym-(F)ICH-Pyr | -Pyr-(F)ICH-Pyr |
| -Diox-(F)ICH-Pyr | -Naf-(F)ICH-Pyr | -$F_2$Phe-(F)ICH-Pyr |
| -(F)Pyr-(F)ICH-Pyr | -(F)Phe-(F)ICH-Pyr | -TDZ-(F)ICH-Pyr |
| -Phe-(F)ICH-Diox | -Pym-(F)ICH-Diox | -Pyr-(F)ICH-Diox |
| -Diox-(F)ICH-Diox | -Naf-(F)ICH-Diox | -$F_2$Phe-(F)ICH-Diox |
| -(F)Pyr-(F)ICH-Diox | -(F)Phe-(F)ICH-Diox | -TDZ-(F)ICH-Diox |
| -Phe-(F)ICH-Naf | -Pym-(F)ICH-Naf | -Pyr-(F)ICH-Naf |
| -Diox-(F)ICH-Naf | -Naf-(F)(CH-Naf | -$F_2$Phe-(F)ICH-Naf |
| -(F)Pyr-(F)ICH-Naf | -(F)Phe-(F)ICH-Naf | -TDZ-(F)ICH-Naf |
| -Phe-(F)ICH-$F_2$Phe | -Pym-(F)ICH-$F_2$Phe | -Pyr-(F)ICH-$F_2$Phe |
| -Diox-(F)ICH-$F_2$Phe | -Naf-(F)ICH-$F_2$Phe | -$F_2$Phe-(F)ICH-$F_2$Phe |
| -(F)Pyr-(F)ICH-$F_2$Phe | -(F)Phe-(F)ICH-$F_2$Phe | -TDZ-(F)ICH-$F_2$Phe |
| -Phe-(F)ICH-(F)Pyr | -Pym-(F)ICH-(F)Pyr | -Pyr-(F)ICH-(F)Pyr |
| -Diox-(F)ICH-(F)Pyr | -Naf-(F)ICH-(F)Pyr | -$F_2$Phe-(F)ICH-(F)Pyr |
| -(F)Pyr-(F)ICH-(F)Pyr | -(F)Phe-(F)ICH-(F)Pyr | -TDZ-(F)ICH-(F)Pyr |
| -Phe-(F)ICH-(F)Phe | -Pym-(F)ICH-(F)Phe | -Pyr-(F)ICH-(F)Phe |
| -Diox-(F)ICH-(F)Phe | -Naf-(F)ICH-(F)Phe | -$F_2$Phe-(F)ICH-(F)Phe |
| -(F)Pyr-(F)ICH-(F)Phe | -(F)Phe-(F)ICH-(F)Phe | -TDZ-(F)ICH-(F)Phe |
| -Phe-(F)ICH-TDZ | -Pym-(F)ICH-TDZ | -Pyr-(F)ICH-TDZ |
| -Diox-(F)ICH-TDZ | -Naf-(F)ICH-TDZ | -$F_2$Phe-(F)ICH-TDZ |
| -(F)Pyr-(F)ICH-TDZ | -(F)Phe-(F)ICH-TDZ | -TDZ-(F)ICH-TDZ |

where the abbreviations have the following meanings:

| | |
|---|---|
| (F)ICH | 1-fluoro-6,7-dihydro-5H-isoquinolin-8-one derivatives, |
| Phe | 1,4-phenylene, |
| Pyr | pyridine-2,5-diyl, |
| Pym | pyrimidine-2,5-diyl, |
| Diox | 1,3-dioxane-2,5-diyl, |
| Naf | naphthalene-2,6-diyl, |
| (F)Phe | fluorobenzene-1,4-diyl |
| $F_2$Phe | difluorobenzene-1,4-diyl, |
| (F)Pyr | fluoropyridine-2,5-diyl and |
| TDZ | 1,3,4-thiadiazole-2,5-diyl |

and $M^1$, $M^6$, $R^1$ and $R^2$ are as defined in the formula (I).

5. The use of a 1-fluoro-6,7-dihydro-5H-isoquinolin-8-one derivative of the formula (I) as claimed in one or more of claims 1 to 4 as a component of a liquid-crystalline mixture.

6. A liquid-crystal mixture comprising one or more compounds of the formula (I) as claimed in one or more of claims 1 to 4.

7. A liquid-crystal mixture as claimed in claim 6, which is ferroelectric.

8. A liquid-crystal mixture as claimed in claim 6 or 7, which comprises from 0.1 to 60 mol% of one or more compounds of the formula (I).

9. A liquid-crystal mixture as claimed in one or more of claims 6 to 8, which comprises from 1 to 10 compounds of the formula (I).

10. A switching and/or display device comprising outer plates, electrodes, at least one polarizer, at least one alignment layer and a liquid-crystalline medium, wherein the liquid-crystalline medium is a liquid-crystal mixture as claimed in one or more of claims 6 to 9.

## Revendications

1. Dérivés de 1-fluoro-6,7-dihydro-5H-isoquinolin-8-one de la formule (I) :

$$R^1(-M^1)_a(-A^1-M^2)_b(-A^2-M^3)_c-B(-M^4-A^3)_d(-M^5-A^4)_e(-M^6)_f-R^2 \qquad (I)$$

dans laquelle les symboles et les indices ont les significations suivantes :
le groupe B est

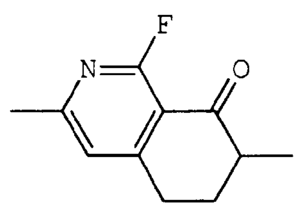

$R^1$, $R^2$ sont identiques ou différents et représentent un hydrogène, -CN, -F, -Cl, $-CF_3$, $-CHF_2$, $-CH_2F$, $-OCF_3$, $-OCHF_2$, $-OCH_2F$ ou un résidu alkyle à chaîne droite ou ramifiée ayant de 1 à 20 atomes de C (avec ou sans atome de C asymétrique), un ou plusieurs groupes CH2 pouvant également être remplacés par -O-, -S-, -CO-O-, -O-CO-, -O-CO-O-, -CO-, -CS-, -CH=CH-, -C≡C-, cyclopropan-1,2-diyle, -Si $(CH_3)_2$-, 1,4-phénylène, trans-1,4-cyclohexylène ou trans-1,3-cyclopentylène, à la condition que des atomes d'oxygène et/ou des atomes de soufre ne puissent pas être liés directement les uns aux autres, et/ou un ou plusieurs atomes de H du résidu alkyle pouvant être substitués par -F, -Cl, -Br, $-OR^3$, -SCN, -OCN ou $-N_3$, mais aussi un des groupes suivants (optiquement actifs ou racémiques) :

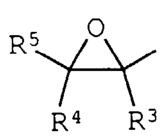

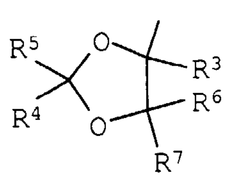

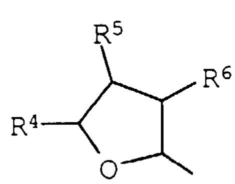

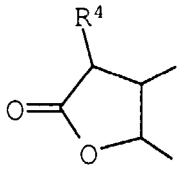
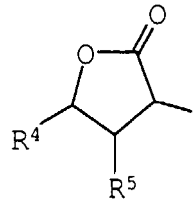
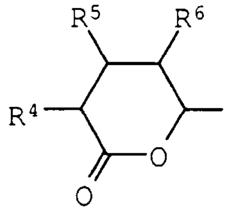

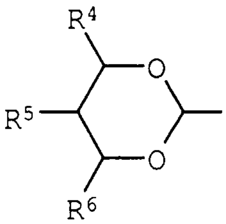
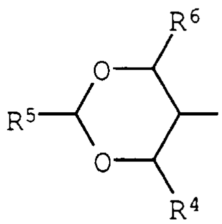
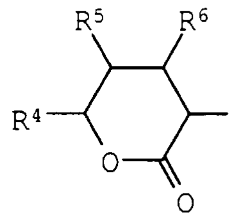

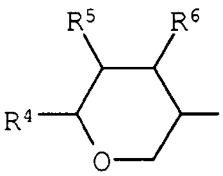
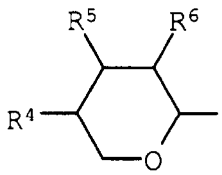
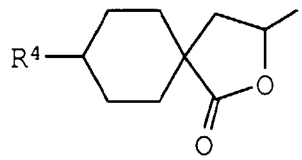

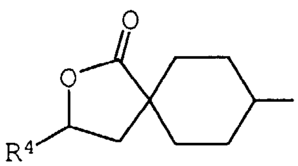
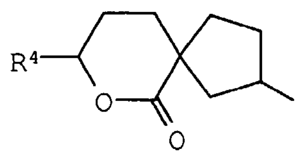
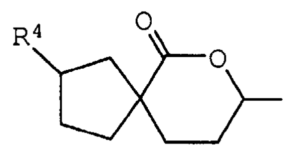

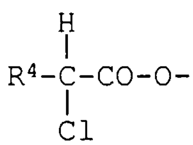
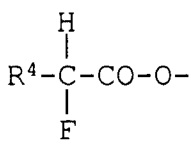
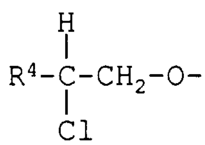
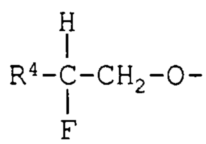

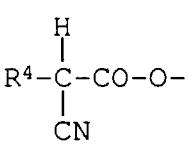
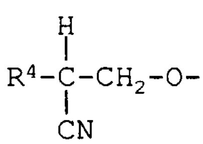
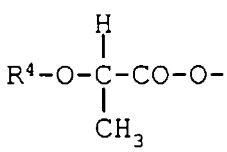
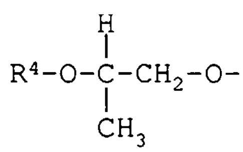

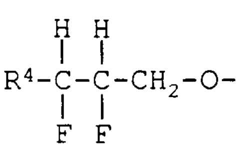
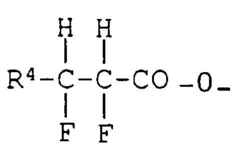

$R^3$, $R^4$, $R^5$, $R^6$, $R^7$ sont identiques ou différents et représentent un hydrogène ou un résidu alkyle à chaîne droite ou ramifiée ayant de 1 à 16 atomes de C (avec ou sans atome de C

asymétrique), un ou plusieurs groupes $CH_2$ pouvant également être remplacés par -O- et/ou -CH=CH-, à la condition que des atomes d'oxygène ne puissent pas être liés directement les uns avec les autres, et/ou un ou plusieurs atomes de H du résidu alkyle pouvant être substitués par -F ou -Cl ; $R^4$ et $R^5$ pris ensemble pouvant également être $-(CH_2)_4-$ ou $-(CH_2)_5-$ lorsqu'ils sont liés à un système oxirane, dioxolane, tétrahydrofurane, tétrahydropyrane, butyrolactone ou valérolactone ;

$M^1$, $M^2$, $M^3$, $M^4$, $M^5$, $M^6$ sont identiques ou différents et représentent -O-, -S-, -CO-, -CO-O-, -O-CO-, -O-CO-O-, -CO-S-, -S-CO-, -CS-O-, -O-CS-, -S-CS-S-, -O-CS-O-, -S-CO-S-, -CS-, $-CH_2-O-$, $-O-CH_2-$, $-CH_2-S-$, $-S-CH_2-$, -CH=CH-, -C≡C-, $-CH_2-CH_2-CO-O$, $-O-CO-CH_2-CH_2-$ ou une liaison simple ;

$A^1$, $A^2$, $A^3$, $A^4$ sont identiques ou différents et représentent un groupe 1,4-phénylène, dans lequel un ou plusieurs atomes de H peuvent être remplacés par F, Cl et/ou CN ; un groupe pyrazin-2,5-diyle, dans lequel un ou deux atomes de H peuvent être remplacés par F, Cl et/ou CN ; un groupe pyridazin-3,6-diyle, dans lequel un ou deux atomes de H peuvent être remplacés par F, Cl et/ou CN ; un groupe pyridin-2,5-diyle, dans lequel un ou plusieurs atomes de H peuvent être remplacés par F, Cl et/ou CN ; un groupe pyrimidin-2,5-diyle, dans lequel un ou deux atomes de H peuvent être remplacés par F, Cl et/ou CN ; un groupe trans-1,4-cyclohexylène, dans lequel un ou deux atomes de H peuvent être remplacés par CN et/ou $CH_3$ ; un groupe (1,3,4)-thiadiazol-2,5-diyle, 1,3-dioxan-2,5-diyle, 1,3-dithian-2,5-diyle, 1,3-thiazol-2,4-diyle, dans lequel un atome de H peut être remplacé par F, Cl et/ou CN ; un groupe 1,3-thiazol-2,5-diyle, dans lequel un atome de H peut être remplacé par F, Cl et/ou CN ; un groupe thiophén-2,4-diyle, dans lequel un atome de H peut être remplacé par F, Cl et/ou CN ; un groupe thiophén-2,5-diyle, dans lequel un ou deux atomes de H peuvent être remplacés par F, Cl et/ou CN ; un groupe pipérazin-1,4-diyle, pipérazin-2,5-diyle, naphtalin-2,6-diyle, dans lequel un ou plusieurs atomes de H peuvent être remplacés par F, Cl et/ou CN ; un groupe bicyclo[2,2,2]octan-1,4-diyle, dans lequel un ou plusieurs atomes de H peuvent être remplacés par F, Cl et/ou CN ; un groupe 1,3-dioxaborinan-2,5-diyle ; ou bien le groupe B ;

a, b, c, d, e, f valent zéro ou un, à la condition que la somme de b, c, d et e soit égale à 0, 1 ou 2.

**2.** Dérivés de 1-fluoro-6,7-dihydro-5H-isoquinolin-8-one selon la revendication 1, caractérisés en ce que, dans la formule (I), les symboles et les indices ont les significations suivantes :

$R^1$, $R^2$ sont identiques ou différents et représentent un hydrogène, -CN, -F, -Cl, $-CF_3$, $-CHF_2$, $-CH_2F$, $-OCF_3$, $-OCHF_2$, -OCH2F ou un résidu alkyle à chaîne droite ou ramifiée ayant de 1 à 18 atomes de C (avec ou sans atome de C asymétrique), un ou plusieurs groupes $CH_2$ pouvant également être remplacés par -O-, -CO-, -CO-O-, -O-CO-, -O-CO-O-, -CH=CH-, -C≡C-, cyclopropan-1,2-diyle, $-Si(CH_3)_2-$ ou trans-1,4-cyclohexylène, à la condition que des atomes d'oxygène ne puissent pas être liés directement les uns aux autres, et/ou un ou plusieurs atomes de H du résidu alkyle pouvant être substitués par -F, -Cl, $-OR^3$, -OCN ou $-N_3$, mais aussi un des groupes suivants (optiquement actifs ou racémiques) :

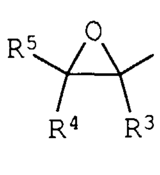
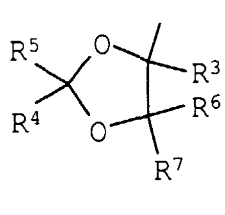
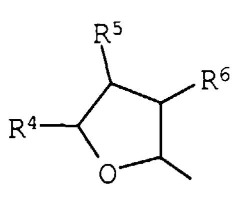

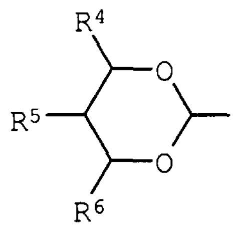 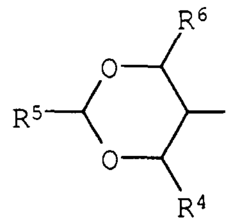 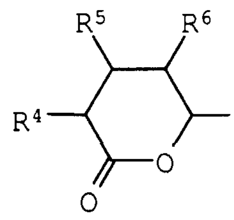

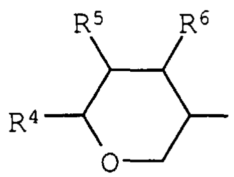 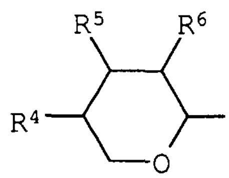 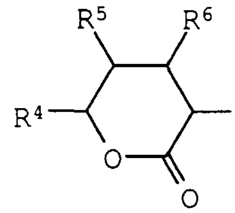

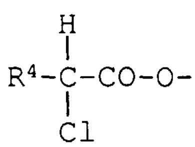 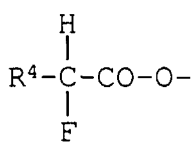 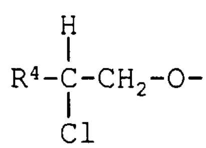 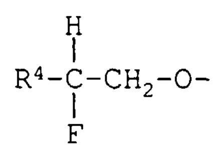

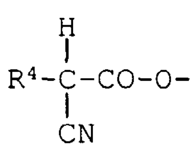 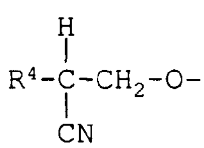 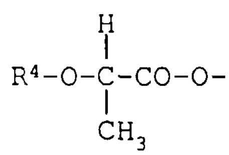 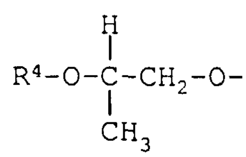

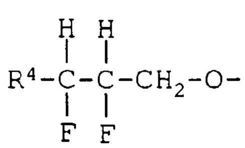 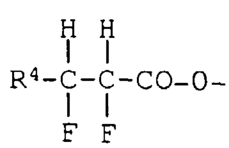

$R^3$, $R^4$, $R^5$, $R^6$, $R^7$ sont identiques ou différents et représentent un hydrogène ou un résidu alkyle à chaîne droite ou ramifiée ayant de 1 à 16 atomes de C (avec ou sans atome de C asymétrique), un ou plusieurs groupes $CH_2$ pouvant également être remplacés par -O- et/ou -CH=CH-, à la condition que des atomes d'oxygène ne puissent pas être liés directement les uns avec les autres, et/ou un ou plusieurs atomes de H du résidu alkyle pouvant être substitués par -F ou -Cl ; $R^4$ et $R^5$ pris ensemble pouvant également être $-(CH_2)_4-$ ou $-(CH_2)_5-$ lorsqu'ils sont liés à un système oxirane, dioxolane, tétrahydrofurane, tétrahydropyrane ou valérolactone ;

$M^1$, $M^2$, $M^3$, $M^4$, $M^5$, $M^6$ sont identiques ou différents et représentent -O-, -CO-, -CO-O-, -O-CO-, -O-CO-O-, -O-CS-O-, $-CH_2-O-$, $O-CH_2-$, -CH=CH-, -C≡C- ou une liaison simple ;

$A^1$, $A^2$, $A^3$, $A^4$ sont identiques ou différents et représentent un groupe 1,4-phénylène, dans lequel un ou plusieurs atomes de H peuvent être remplacés par F, Cl et/ou CN ; un groupe pyrazin-2,5-diyle, dans lequel un ou deux atomes de H peuvent être remplacés par F, Cl et/ou CN ; un groupe pyridazin-3,6-diyle, dans lequel un ou deux atomes de H peuvent être remplacés par F, Cl et/ou CN ; un groupe pyridin-2,5-diyle, dans lequel un ou plusieurs atomes de H peuvent être remplacés par F, Cl et/ou CN ; un groupe pyrimidin-2,5-diyle, dans lequel un ou deux atomes de H peuvent être remplacés par F, Cl et/ou CN ; un groupe trans-1,4-cyclohexylène, dans lequel un ou

deux atomes de H peuvent être remplacés par CN et/ou $CH_3$ ; un groupe (1,3,4)-thiadiazol-2,5-diyle, 1,3-dioxan-2,5-diyle, thiophén-2,4-diyle, dans lequel un atome de H peut être remplacé par F, Cl et/ou CN ; un groupe thiophén-2,5-diyle, dans lequel un ou deux atomes de H peuvent être remplacés par F, Cl et/ou CN ; un groupe naphtalin-2,6-diyle, dans lequel un ou plusieurs atomes de H peuvent être remplacés par F, Cl et/ou CN ; ou bien le groupe B ;

a, b, c, d, e, f — valent zéro ou un, à la condition que la somme de b, c, d et e soit égale à 0, 1 ou 2.

**3.** Dérivés de 1-fluoro-6,7-dihydro-5H-isoquinolin-8-one selon la revendication 1 et/ou la revendication 2, caractérisés en ce que, dans la formule (I), les symboles et les indices ont les significations suivantes :

$R^1$, $R^2$ — sont identiques ou différents et représentent un hydrogène, -CN, -F, -Cl, $-CF_3$, $-CHF_2$, $-CH_2F$, $-OCF_3$, $-OCHF_2$, $-OCH_2F$ ou un résidu alkyle à chaîne droite ou ramifiée ayant de 1 à 16 atomes de C (avec ou sans atome de C asymétrique), un, deux ou trois groupes CH2 pouvant également être remplacés par -O-, -CO-, -O-CO-, -CO-O-, -CH=CH-, cyclopropan-1,2-diyle, $-Si(CH_3)_2-$ ou trans-1,4-cyclohexylène, à la condition que des atomes d'oxygène ne puissent pas être liés directement les uns aux autres, et/ou un ou plusieurs atomes de H du résidu alkyle pouvant être substitués par -F, -Cl ou $-OR^3$, mais aussi un des groupes suivants (optiquement actifs ou racémiques) :

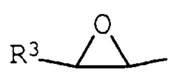
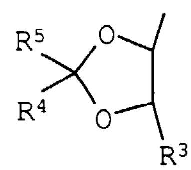
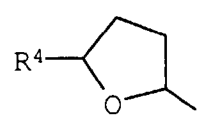

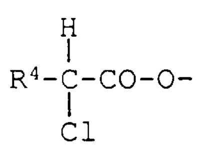
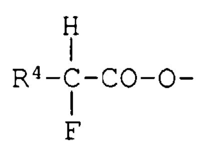
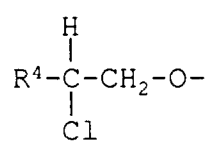
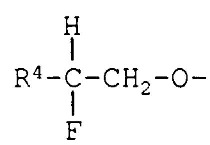

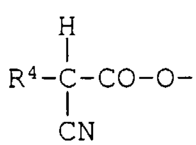
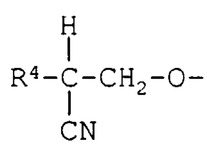
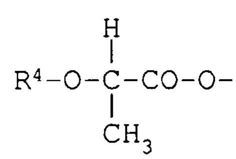
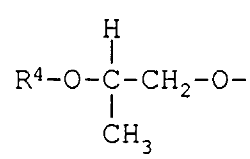

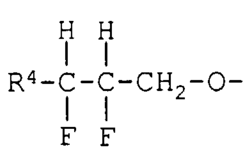
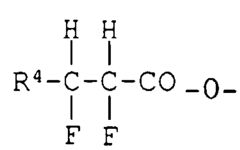

$R^3$, $R^4$, $R^5$ — sont identiques ou différents et représentent un hydrogène ou un résidu alkyle à chaîne droite ou ramifiée ayant de 1 à 9 atomes de C (avec ou sans atome de C asymétrique), un ou plusieurs groupes $CH_2$ pouvant également être remplacés par -O- et/ou -CH=CH-, à la condition que des atomes d'oxygène ne puissent pas être liés directement les uns avec les autres, et/ou un ou plusieurs atomes de H du résidu alkyle pouvant être substitués par -F ou -Cl ; $R^4$ et $R^5$ pris ensemble pouvant également être $-(CH_2)_4-$ ou $-(CH_2)_5-$ lorsqu'ils sont liés à un système dioxolane ;

$M^1$, $M^2$, $M^3$, $M^4$, $M^5$, $M^6$ — sont identiques ou différents et représentent -O-, -CO-, -CO-O-, -O-CO-, -O-CO-O-,

| | |
|---|---|
| | $-CH_2-O-$, $O-CH_2-$, -CH=CH- ou une liaison simple ; |
| $A^1$, $A^2$, $A^3$, $A^4$ | sont identiques ou différents et représentent un groupe 1,4-phénylène, dans lequel un, deux ou trois atomes de H peuvent être remplacés par F, Cl et/ou CN ; un groupe pyridin-2,5-diyle, dans lequel un ou deux atomes de H peuvent être remplacés par F, Cl et/ou CN ; un groupe pyrimidin-2,5-diyle, dans lequel un ou deux atomes de H peuvent être remplacés par F, Cl et/ou CN ; un groupe trans-1,4-cyclohexylène, dans lequel un ou deux atomes de H peuvent être remplacés par CN et/ou $CH_3$ ; un groupe (1,3,4)-thiadiazol-2,5-diyle ou naphtalin-2,6-diyle, dans lequel un ou deux atomes de H peuvent être remplacés par F, Cl et/ou CN ; |
| a, b, c, d, e, f | valent zéro ou un, à la condition que la somme de b, c, d et e soit égale à 0, 1 ou 2. |

**4.** Dérivés de 1-fluoro-6,7-dihydro-5H-isoquinolin-8-one selon l'une ou plusieurs des revendications 1 à 3, caractérisés en ce que le groupe $(-A^1-M^2)_b(-A^2-M^3)_c-B(-M^4-A^3)_d(-M^5-A^4)_e$ a l'une des significations suivantes :

| | | |
|---|---|---|
| -Phe-Phe-(F)ICH | -Phe-Pym-(F)ICH | -Phe-Pyr-(F)ICH |
| -Phe-Diox-(F)ICH | -Phe-Naf-(F)ICH | -Phe-$F_2$Phe-(F)ICH |
| -Phe-(F)Pyr-(F)ICH | -Phe-(F)Phe-(F)ICH | -Phe-TDZ-(F)ICH |
| -Pym-Phe-(F)ICH | -Pym-Pym-(F)ICH | -Pym-Pyr-(F)ICH |
| -Pym-Diox-(F)ICH | -Pym-Naf-(F)ICH | -Pym-$F_2$Phe-(F)ICH |
| -Pym-(F)Pyr-(F)ICH | -Pym-(F)Phe-(F)ICH | -Pym-TDZ-(F)ICH |
| -Pyr-Phe-(F)ICH | -Pyr-Pym-(F)ICH | -Pyr-Pyr-(F)ICH |
| -Pyr-Diox-(F)ICH | -Pyr-Naf-(F)ICH | -Pyr-$F_2$Phe-(F)ICH |
| -Pyr-(F)Pyr-(F)ICH | -Pyr-(F)Phe-(F)ICH | -Pyr-TDZ-(F)ICH |
| -Diox-Phe-(F)ICH | -Diox-Pym-(F)ICH | -Diox-Pyr-(F)ICH |
| -Diox-Diox-(F)ICH | -Diox-Naf-(F)ICH | -Diox-$F_2$Phe-(F)ICH |
| -Diox-(F)Pyr-(F)ICH | -Diox-(F)Phe-(F)ICH | -Diox-TDZ-(F)ICH |
| -Naf-Phe-(F)ICH | -Naf-Pym-(F)ICH | -Naf-Pyr-(F)ICH |
| -Naf-Diox-(F)ICH | -Naf-Naf-(F)ICH | -Naf-$F_2$Phe-(F)ICH |
| -Naf-(F)Pyr-(F)ICH | -Naf-(F)Phe-(F)ICH | -Naf-TDZ-(F)ICH |
| -$F_2$Phe-Phe-(F)ICH | -$F_2$Phe-Pym-(F)ICH | -$F_2$Phe-Pyr-(F)ICH |
| -$F_2$Phe-Diox-(F)ICH | -$F_2$Phe-Naf-(F)ICH | -$F_2$Phe-$F_2$Phe-(F)ICH |
| -$F_2$Phe-(F)Pyr-(F)ICH | -$F_2$Phe-(F)Phe-(F)ICH | -$F_2$Phe-TDZ-(F)ICH |
| -(F)Pyr-Phe-(F)ICH | -(F)Pyr-Pym-(F)ICH | -(F)Pyr-Pyr-(F)ICH |
| -(F)Pyr-Diox-(F)ICH | -(F)Pyr-Naf-(F)ICH | -(F)Pyr-$F_2$Phe-(F)ICH |
| -(F)Pyr-(F)Pyr-(F)ICH | -(F)Pyr-(F)Phe-(F)ICH | -(F)Pyr-TDZ-(F)ICH |
| -(F)Phe-Phe-(F)ICH | -(F)Phe-Pym-(F)ICH | -(F)Phe-Pyr-(F)ICH |
| -(F)Phe-Diox-(F)ICH | -(F)Phe-Naf-(F)ICH | -(F)Phe-$F_2$Phe-(F)ICH |
| -(F)Phe-(F)Pyr-(F)ICH | -(F)Phe-(F)Phe-(F)ICH | -(F)Phe-TDZ-(F)ICH |
| -TDZ-Phe-(F)ICH | -TDZ-Pym-(F)ICH | -TDZ-Pyr-(F)ICH |
| -TDZ-Diox-(F)ICH | -TDZ-Naf-(F)ICH | -TDZ-$F_2$Phe-(F)ICH |
| -TDZ-(F)Pyr-(F)ICH | -TDZ-(F)Phe-(F)ICH | -TDZ-TDZ-(F)ICH |
| -Phe-(F)ICH | -Pym-(F)ICH | -Pyr-(F)ICH |
| -Diox-(F)ICH | -Naf-(F)ICH | -$F_2$Phe-(F)ICH |
| -(F)Pyr-(F)ICH | -(F)Phe-(F)ICH | -TDZ-(F)ICH |
| -(F)ICH-Phe-Phe | -(F)ICH-Pym-Phe | -(F)ICH-Pyr-Phe |
| -(F)ICH-Diox-Phe | -(F)ICH-Naf-Phe | -(F)ICH-$F_2$Phe-Phe |
| -(F)ICH-(F)Pyr-Phe | -(F)ICH-(F)Phe-Phe | -(F)ICH-TDZ-Phe |
| -(F)ICH-Phe-Pym | -(F)ICH-Pym-Pym | -(F)ICH-Pyr-Pym |
| -(F)ICH-Diox-Pym | -(F)ICH-Naf-Pym | -(F)ICH-$F_2$Phe-Pym |
| -(F)ICH-(F)Pyr-Pym | -(F)ICH-(F)Phe-Pym | -(F)ICH-TDZ-Pym |
| -(F)ICH-Phe-Pyr | -(F)ICH-Pym-Pyr | -(F)ICH-Pyr-Pyr |
| -(F)ICH-Diox-Pyr | -(F)ICH-Naf-Pyr | -(F)ICH-$F_2$Phe-Pyr |
| -(F)ICH-(F)Pyr-Pyr | -(F)ICH-(F)Phe-Pyr | -(F)ICH-TDZ-Pyr |

(suite)

| | | |
|---|---|---|
| -(F)ICH-Phe-Diox | -(F)ICH-Pym-Diox | -(F)ICH-Pyr-Diox |
| -(F)ICH-Diox-Diox | -(F)ICH-Naf-Diox | -(F)ICH-$F_2$Phe-Diox |
| -(F)ICH-(F)Pyr-Diox | -(F)ICH-(F)Phe-Diox | -(F)ICH-TDZ-Diox |
| -(F)ICH-Phe-Naf | -(F)ICH-Pym-Naf | -(F)ICH-Pyr-Naf |
| -(F)ICH-Diox-Naf | -(F)ICH-Naf-Naf | -(F)ICH-$F_2$Phe-Naf |
| -(F)ICH-(F)Pyr-Naf | -(F)ICH-(F)Phe-Naf | -(F)ICH-TDZ-Naf |
| -(F)ICH-Phe-$F_2$Phe | -(F)ICH-Pym-$F_2$Phe | -(F)ICH-Pyr-$F_2$Phe |
| -(F)ICH-Diox-$F_2$Phe | -(F)ICH-Naf-$F_2$Phe | -(F)ICH-$F_2$Phe-$F_2$Phe |
| -(F)ICH-(F)Pyr-$F_2$Phe | -(F)ICH-(F)Phe-$F_2$Phe | -(F)ICH-TDZ-$F_2$Phe |
| -(F)ICH-Phe-(F)Pyr | -(F)ICH-Pym-(F)Pyr | -(F)ICH-Pyr-(F)Pyr |
| -(F)ICH-Diox-(F)Pyr | -(F)ICH-Naf-(F)Pyr | -(F)ICH-$F_2$Phe-(F)Pyr |
| -(F)ICH-(F)Pyr-(F)Pyr | -(F)ICH-(F)Phe-(F)Pyr | -(F)ICH-TDZ-(F)Pyr |
| -(F)ICH-Phe-(F)Phe | -(F)ICH-Pym-(F)Phe | -(F)ICH-Pyr-(F)Phe |
| -(F)ICH-Diox-(F)Phe | -(F)ICH-Naf-(F)Phe | -(F)ICH-$F_2$Phe-(F)Phe |
| -(F)ICH-(F)Pyr-(F)Phe | -(F)ICH-(F)Phe-(F)Phe | -(F)ICH-TDZ-(F)Phe |
| -(F)ICH-Phe-TDZ | -(F)ICH-Pym-TDZ | -(F)ICH-Pyr-TDZ |
| -(F)ICH-Diox-TDZ | -(F)ICH-Naf-TDZ | -(F)ICH-$F_2$Phe-TDZ |
| -(F)ICH-(F)Pyr-TDZ | -(F)ICH-(F)Phe-TDZ | -(F)ICH-TDZ-TDZ |
| -(F)ICH-Phe | -(F)ICH-Pym | -(F)ICH-Pyr |
| -(F)ICH-Diox | -(F)ICH-Naf | -(F)ICH-$F_2$Phe |
| -(F)ICH-(F)Pyr | -(F)ICH-(F)Phe | -(F)ICH-TDZ |
| -Phe-(F)ICH-Phe | -Pym-(F)ICH-Phe | -Pyr-(F)ICH-Phe |
| -Diox-(F)ICH-Phe | -Naf-(F)ICH-Phe | -$F_2$Phe-(F)ICH-Phe |
| -(F)Pyr-(F)ICH-Phe | -(F)Phe-(F)ICH-Phe | -TDZ-(F)ICH-Phe |
| -Phe-(F)ICH-Pym | -Pym-(F)ICH-Pym | -Pyr-(F)ICH-Pym |
| -Diox-(F)ICH-Pym | -Naf-(F)ICH-Pym | -$F_2$Phe-(F)ICH-Pym |
| -(F)Pyr-(F)ICH-Pym | -(F)Phe-(F)ICH-Pym | -TDZ-(F)ICH-Pym |
| -Phe-(F)ICH-Pyr | -Pym-(F)ICH-Pyr | -Pyr-(F)ICH-Pyr |
| -Diox-(F)ICH-Pyr | -Naf-(F)ICH-Pyr | -$F_2$Phe-(F)ICH-Pyr |
| -(F)Pyr-(F)ICH-Pyr | -(F)Phe-(F)ICH-Pyr | -TDZ-(F)ICH-Pyr |
| -Phe-(F)ICH-Diox | -Pym-(F)ICH-Diox | -Pyr-(F)ICH-Diox |
| -Diox-(F)ICH-Diox | -Naf-(F)ICH-Diox | -$F_2$Phe-(F)ICH-Diox |
| -(F)Pyr-(F)ICH-Diox | -(F)Phe-(F)ICH-Diox | -TDZ-(F)ICH-Diox |
| -Phe-(F)ICH-Naf | -Pym-(F)ICH-Naf | -Pyr-(F)ICH-Naf |
| -Diox-(F)ICH-Naf | -Naf-(F)ICH-Naf | -$F_2$Phe-(F)ICH-Naf |
| -(F)Pyr-(F)ICH-Naf | -(F)Phe-(F)ICH-Naf | -TDZ-(F)ICH-Naf |
| -Phe-(F)ICH-$F_2$Phe | -Pym-(F)ICH-$F_2$Phe | -Pyr-(F)ICH-$F_2$Phe |
| -Diox-(F)ICH-$F_2$Phe | -Naf-(F)ICH-$F_2$Phe | -$F_2$Phe-(F)ICH-$F_2$Phe |
| -(F)Pyr-(F)ICH-$F_2$Phe | -(F)Phe-(F)ICH-$F_2$Phe | -TDZ-(F)ICH-$F_2$Phe |
| -Phe-(F)ICH-(F)Pyr | -Pym-(F)ICH-(F)Pyr | -Pyr-(F)ICH-(F)Pyr |
| -Diox-(F)ICH-(F)Pyr | -Naf-(F)ICH-(F)Pyr | -$F_2$Phe-(F)ICH-(F)Pyr |
| -(F)Pyr-(F)ICH-(F)Pyr | -(F)Phe-(F)ICH-(F)Pyr | -TDZ-(F)ICH-(F)Pyr |
| -Phe-(F)ICH-(F)Phe | -Pym-(F)ICH-(F)Phe | -Pyr-(F)ICH-(F)Phe |
| -Diox-(F)ICH-(F)Phe | -Naf-(F)ICH-(F)Phe | -$F_2$Phe-(F)ICH-(F)Phe |
| -(F)Pyr-(F)ICH-(F)Phe | -(F)Phe-(F)ICH-(F)Phe | -TDZ-(F)ICH-(F)Phe |
| -Phe-(F)ICH-TDZ | -Pym-(F)ICH-TDZ | -Pyr-(F)ICH-TDZ |
| -Diox-(F)ICH-TDZ | -Naf-(F)ICH-TDZ | -$F_2$Phe-(F)ICH-TDZ |
| -(F)Pyr-(F)ICH-TDZ | -(F)Phe-(F)ICH-TDZ | -TDZ-(F)ICH-TDZ |

les abréviations ayant les significations suivantes :

| | |
|---|---|
| (F)ICH = | 1-fluoro-6,7-dihydro-5H-isoquinolin-8-on-3,7-diyle, |
| Phe = | 1,4-phénylène, |
| Pyr = | pyridin-2,5-diyle, |
| Pym = | pyrimidin-2,5-diyle, |
| Diox = | 1,3-dioxan-2,5-diyle, |
| Naf = | naphtalin-2,6-diyle, |
| $F_2$Phe = | difluorobenzén-1,4-diyle, |
| (F)Phe = | fluorobenzén-1,4-diyle, |
| (F)Pyr = | fluoropyridin-2,5-diyle et |
| TDZ = | (1,3,4)-thiadiazol-2,5-diyle, |

et $M^1$, $M^6$, $R^1$, $R^2$ ont les significations indiquées dans la formule (I).

**5.** Utilisation de dérivés de 1-fluoro-6,7-dihydro-5H-isoquinolin-8-one de la formule (I) selon l'une ou plusieurs des revendications 1 à 4 comme composants de mélanges à cristaux liquides.

**6.** Mélange à cristaux liquides contenant un ou plusieurs composés de la formule (I) selon l'une ou plusieurs des revendications 1 à 4.

**7.** Mélange à cristaux liquides selon la revendication 6, caractérisé en ce qu'il est ferroélectrique.

**8.** Mélange à cristaux liquides selon la revendication 6 ou la revendication 7, caractérisé en ce qu'il contient de 0,1 à 60 % molaires d'un ou plusieurs des composés de la formule (I).

**9.** Mélange à cristaux liquides selon l'une ou plusieurs des revendications 6 à 8, caractérisé en ce qu'il contient de 1 à 10 composés de la formule (I).

**10.** Dispositif de commutation et/ou d'affichage contenant des plaques supports, des électrodes, au moins un polariseur, au moins une couche d'orientation ainsi qu'un milieu à cristaux liquides, caractérisé en ce que le milieu à cristaux liquides est un mélange à cristaux liquides selon l'une ou plusieurs des revendications 6 à 9.